# EUROPEAN PATENT APPLICATION

(11) **EP 4 385 997 A1**
(43) Date of publication of application: **19.06.2024**
(21) Application number: 22214372.9
(22) Date of filing: 16.12.2022
(51) Int. Cl.: C07K 7/64, A01N 37/46

(54) **CHITINOPEPTINS, THEIR USE AND RECOMBINANT SYNTHESIS IN ORGANISMS FOR PLANT PROTECTION**

(71) Applicant: Fraunhofer-Gesellschaft zur Förderung der angewandten Forschung e.V., 80686 München (DE)
(72) Inventor: MARNER, Michael, 35440 Linden (DE); SPOHN, Marius, 35423 Lich (DE); SCHÄBERLE, Till, 35394 Gießen (DE); PATRAS, Maria, 35444 Biebertal (DE); MIHAJLOVIC, Sanja, 35418 Buseck (DE)
(74) Representative: Kuttenkeuler, David

(57) **Abstract**

The invention is based on an unexpected antimicrobial effect of Chitinopeptin A and its iron(III) complex against the Gram-negative bacterium *Xylella fastidiosa.* In a first aspect, the invention pertains to a peptide comprising a chain of amino acids as shown in this document, and in a second aspect to a complex of said peptide. In further aspects, the invention pertains to a nucleic acid encoding for the peptide, a non-ribosomal peptide synthetase (NRPS), a vector and a cell comprising the previously mentioned subject matter. Additionally, the invention pertains to a plant protection agent and a method of manufacturing the aforementioned subject matter. Based on the unexpected antimicrobial effect of Chitinopeptin A, furthermore a use of the peptide, a plant protection agent, a plant protection composition and a method for the treatment of a disease and/or increasing resistance against infection in a plant is disclosed.

## Description

### FIELD OF THE INVENTION

The invention is based on an unexpected antimicrobial effect of Chitinopeptin A and its iron(III) complex against the Gram-negative bacterium *Xylella fastidiosa.* In a first aspect, the invention pertains to a peptide comprising a chain of amino acids as shown in this document, and in a second aspect to a complex of said peptide. In further aspects, the invention pertains to a nucleic acid encoding for the peptide, a non-ribosomal peptide synthetase (NRPS), a vector and a cell comprising the previously mentioned subject matter. Additionally, the invention pertains to a plant protection agent and a method of manufacturing the aforementioned subject matter. Based on the unexpected antimicrobial effect of Chitinopeptin A, furthermore a use of the peptide, a plant protection agent, a plant protection composition and a method for the treatment of a disease and/or increasing resistance against infection in a plant is disclosed.

### DESCRIPTION

At the COP26 climate change conference in Glasgow, world leaders agreed that protecting and restoring forests is key to achieve the United Nations sustainability goals. As a result, the Glasgow COP26 Declaration on Forest and Land use was signed by 141 countries, including Germany, China and the USA. At the same time, global forest cover is facing a devastating plant epidemic caused by the Gram-negative bacterium *Xylellafastidiosa.*

The quarantine pathogen *Xylella* is one of the deadliest pathogens in plants. *Xylella* has an extremely wide host range and affects >600 cultivated and wild plant species. In addition to almond trees, grapes (Pierce's disease), olive trees (olive quick decline syndrome) and citrus plants (citrus variegated chlorosis), *Xylella* also attacks deciduous trees such as elms, maples and oaks (1). Symptoms include tissue desiccation, wilting of branches and leaves, and eventual death of the infected plant. The destruction of long-lived, woody plants leads to a particularly high economic and (socio-) ecological loss.

*Xylella* is transmitted by xylem-sucking insects and can thus rapidly spread over large spread distances. In vector insects, *Xylella* colonizes the oral cavity and is injected into healthy plants during the sucking process. In the plant, the pathogen reduces the performance of the vascular system and thereby impairs water and nutrient distribution. The consequences of climate change are accelerating the global spread of *Xylella fastidiosa.* Outbreaks were first reported in North and South America, but since 2013 the pathogen has also been wreaking havoc in the Mediterranean region.

There is a great need for strategies to prevent further progression of the destruction of wild and cultivated plants caused by *Xylella fastidiosa.* There is currently no solution on the market to combat *Xylella fastidiosa* infection and there is no cure for already infected forests. Neither strict control and quarantine of imported plants nor the cultivation of less susceptible cultivars has been able to reliably contain the spread of *Xylella fastidiosa.* The result is extensive clearing of infected and adjacent forest areas.

Preventive measures against vector insect-mediated infectious diseases are mostly synthetic broad-spectrum pesticides. These substances are hardly biodegradable and spread, among other things, via rainwater. They are detectable in toxic concentrations in groundwater and soil. (2,3) The long-term effect on humans and the environment is worrying. The ban of these products from agriculture (e.g. insect protection law) must be compensated for by the introduction of alternative solutions.

In US20050053584A1 (4) and US20180310569A1 (5), the avirulent strain *X. fastidiosa* EB92-1 ("benign" strain) has been disclosed as a biocontrol strainto eliminate virulent *Xylella strains* in the plant through growth competition. However, it can be assumed that the missing virulence factors represent a competitive disadvantage. A preventive establishment of the benign strain in the plant could compensate for this disadvantage, whereby the effect would have to be refreshed regularly ("booster administration").

Further experimental approaches to treat already infected plants include phage cocktails (6); Xylem endophytes (7, 8), application of bactericidal metals (9), application of bactericidal metal complexes (10) and the stimulation of plant-specific defenses. However, so far none of these research approaches has shown any effectiveness in field trials. (11)

Dentamet^{®} (copper-zinc citric acid biocomplex) is currently being investigated as a fertilizer against *Xylella* infections. (12) Copper and zinc ions are taken up by the plant and interfere with the growth of *Xylella* in the plant. Long-term studies are pending.

*Xylella* inhibitory effects have been observed in experiments with synthetic and naturally occurring anti-microbial peptides (AMPs). (13-17) High production costs (compared to small molecules) (18) and an often unspecific mode of action have so far prevented development in the agricultural industry. (19) In further approaches, plants (seeds) (20) or avirulent plant viruses (21) are genetically modified to enable the transgenic expression of AMPs and thus protect the plant from infectious diseases. Furthermore, genetically modified endophytes or plants can express specific genes of *Xylella* sp. (e.g. HecA-like hemagglutinin gene) to slow down or prevent spread of the pathogen in the plant. (22). However, approval for the cultivation of genetically modified plants is lengthy and has not yet been granted for the plants concerned. In addition, acceptance of genetically modified plants is low, particularly in the EU, and approval of such plants is not to be expected in the short term.

In addition, *Xylella* can be killed by a number of classic antibiotics. (23) However, the use of clinical antibiotics in plants is banned in the EU to prevent the development of resistance.

Furthermore, microbial natural products (not used in pharmaceuticals) are an attractive source of new active ingredients to combat *Xylella fastistiosa.* Radicinin (24, 25), a known substance from various plant-associated fungi, inactivates serine proteases from Xylella and thus inhibits the growth of the bacterium. *Xylella* inhibitory effects have been observed for other fungal metabolites (e.g. ophiobilins (26) and gliotoxins). Toxic, especially phytotoxic, properties prevent its use as a crop protection agent. (27,28)

Natural Product (NP) production is an adaptive mechanism providing evolutionary fitness upon changing environmental conditions and in the presence of growth competitors for the respective producer organism. (29) In a recent study (30), the inventors mapped 600 genomes of phylum *Bacteroidetes* to systematically access their biosynthetic potential.

Due to a high abundance and diversity of biosynthetic gene clusters, the genus *Chitinophaga* was selected for performing a bioactivity-guided NP discovery program.

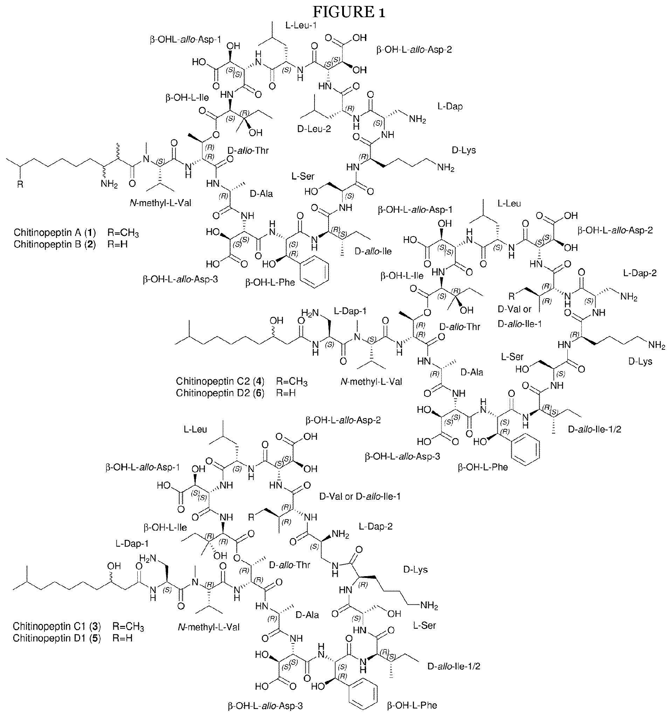

Bioactivity-guided fractionation by UHPLC-HR-ESI-MS led to the identification of six cyclic lipodepsipeptides. *C. eiseniae* produced the two tetradecalipodepsipeptides Chitinopeptin A and Chitinopeptin B, whereas *C. flava* assembled the four pentadecalipodepsipeptides Chitinopeptin C1 and Chitinopeptin C2 and Chitinopeptin D1 + Chitinopeptin D2 with the molecular structures shown in Figure 1. Additional experiments revealed that the Chitinopeptins have an antimicrobial effect on Gram-negative and Gram-positive bacteria, as well as against filamentous fungi and *C. albicans.*

Additionally, the inventors scanned the genomes of *C. eiseniae* (FUWZ01.1) and *C. flava* (QFFJ01.1) for NRPS-type BGCs matching the structural features of Chitinopeptins and identified BGCs in *C. eiseniae* and *C. flava* congruent to the cyclic lipopetpides structure in each case. Furthermore, they confirmed two further related BGCs within the genomes of *C. oryziterrae* JCM16595 (WRXO01.1) and *C. niastensis* DSM 24859 (PYAW01.1).

As mentioned above, there is no adequate strategy to counter the spreading of *Xylella fastidiosa* in plants. Herein, the inventors show yet unpublished results that demonstrate a previously unknown antimicrobial effect of Chitinopeptin A on *Xylella fastidiosa.* The inventors were able to show the efficacy of this molecule both in an unbound, as well as in a complex with iron. Thus, it is an object of the invention to provide compounds (such as a peptide and a complex), which can be used for the protection or curation of plants against *Xylella fastidiosa.* The inventors provide a method for treating *Xylella fastidiosa*-based infections and/or increasing the resistance against such infections in a plant and means for the manufacturing of the peptide and the complex and plant protection agents containing these compounds.

### BRIEF DESCRIPTION OF THE INVENTION

Generally, and by way of brief description, the main aspects of the present invention can be described as follows:

**In the first aspect,** the invention pertains to a peptide that comprises a chain of amino acids with the sequence X₁X₂X₃X₄X₅X₆X₇X₈X₉X₁₀X₁₁X₁₂X₁₃X₁₄X₁₅;
wherein X is an amino acid or no amino acid (absent) according to the following definitions:
- X₁ is absent or L-Dap;
- X₂ is N-methyl-L-Val;
- X₃ is D-*allo*-Thr;
- X₄ is D-Ala;
- X₅ is β-OH-L-*allo*-Asp;
- X₆ is β-OH-L-Phe;
- X₇ is D-*allo*-Ile;
- X₈ is L-Ser;
- X₉ is D-Lys;
- X₁₀ is L-Dap;
- X₁₁ is D-Val, D-*allo*-Ile or D-Leu;
- X₁₂ is β-OH-L-*allo*-Asp;
- X₁₃ is L-Leu;
- X₁₄ is β-OH-L-*allo*-Asp;
- X₁₅ is β-OH-L-Ile;
   wherein L-Dap is L-2,3-Diaminopropionic acid;
   wherein sequential amino acids are connected by a carboxamide bond, and wherein a fatty acid group R is bound covalently to an amino acid of the chain of amino acids of the peptide,
   wherein the amino acids X₃X₄X₅X₆X₇X₈X₉X₁₀X₁₁X₁₂X₁₃X₁₄X₁₅ of the peptide form a cycle and the non-side chain carboxyl group of the amino acid X₁₅ is covalently connected to the amino acid X₃.

**In a second aspect,** the invention pertains to a complex consisting of a peptide according any of the other aspects of the invention and a metal atom.

**In a third aspect,** the invention pertains to a nucleic acid sequence encoding for the peptide according to any of the aspects of the invention.

**In a fourth aspect,** the invention pertains to an NRPS that is able to synthesise the peptide according to any one of the other aspects of the invention.

**In a fifth aspect,** the invention pertains to a vector comprising a nucleic acid sequence according to the any aspect of the invention.

**In a sixth aspect,** the invention pertains to a cell comprising the peptide, the complex, the nucleic acid sequence, the NRPS, and/or the vector according to one of the other aspects of the invention.

**In a seventh aspect,** the invention pertains to a plant protection agent comprising the peptide, the complex, a nucleic acid sequence, the NRPS, the vector and/or the cell according to any one of the other aspects of the invention.

**In an eighth aspect,** the invention pertains to a method of manufacturing a peptide, the complex, the NRPS and/or the plant protection agent according to any aspect of the invention, wherein the method comprises a recombinant and/or a chemical synthesis.

**In a ninth aspect,** the invention pertains to a plant protection agent, obtainable by the method according to any one of other aspects of the invention.

**In a tenth aspect,** the invention pertains to a method for treating a disease and/or increasing resistance against an infection in a plant, wherein method comprises administering to the plant a peptide, complex and/or plant protection agent according to any one of the other aspects of the invention.

**In an eleventh aspect,** the invention pertains to a use of a plant protection agent according to any aspect of the invention in the treatment of a disease and/or increasing resistance against infection in a plant.

**In a twelfth aspect,** the invention pertains to a plant protection composition comprising at least one peptide or a salt thereof according to any one of the other aspects of the invention and a phytopharmaceutically acceptable carrier and/or excipient.

**In a thirteenth aspect,** the invention pertains to the use of a peptide according to any other aspect of the invention as a plant protection agent.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **the first aspect,** the invention pertains to a peptide that comprises a chain of amino acids with the sequence X₁X₂X₃X₄X₅X₆X₇X₈X₉X₁₀X₁₁X₁₂X₁₃X₁₄X₁₅ **(SEQ ID NO.1**);
wherein X is an amino acid or no amino acid (absent) according to the following definitions:
- X₁ is absent or L-Dap;
- X₂ is N-methyl-L-Val;
- X₃ is D-*allo*-Thr;
- X₄ is D-Ala;
- X₅ is β-OH-L-*allo*-Asp;
- X₆ is β-OH-L-Phe;
- X₇ is D-*allo*-Ile;
- X₈ is L-Ser;
- X₉ is D-Lys;
- X₁₀ is L-Dap;
- X₁₀ is D-Val, D-*allo*-Ile or D-Leu;
- X₁₂ is β-OH-L-*allo*-Asp;
- X₁₃ is L-Leu;
- X₁₄ is β-OH-L-*allo*-Asp;
- X₁₅ is β-OH-L-Ile;
   wherein L-Dap is L-2,3-Diaminopropionic acid;
   wherein sequential amino acids are connected by a carboxamide bond, and wherein a fatty acid group R is bound covalently to an amino acid of the chain of amino acids of the peptide;
   wherein the amino acids X₃X₄X₅X₆X₇X₈X₉X₁₀X₁₁X₁₂X₁₃X₁₄X₁₅ **(SEQ ID NO. 2**) of the peptide form a cycle and the non-side chain carboxyl group of the amino acid X₁₅ is covalently connected to the amino acid X₃.

In preferred embodiments of the invention, the chain of amino acids is:
X₁~X₂~X₃~X₄~X₅~X₆~X₇~X₈~X₉^{∗}X₁₀~X₁₁~X₁₂~X₁₃~X₁₄~X₁₅ **(SEQ ID NO. 3 and 4**) or
X₁~X₂~X₃~X₄~X₅~X₆~X₇~X₈~X₉~X₁₀~X₁₁~X₁₂~X₁₃~X₁₄~X₁₅ (**SEQ ID NO. 5**);
wherein ~ between two adjacent amino acids indicates that the two adjacent amino acids are covalently connected by a peptide bond connecting the non-side chain carboxyl group of the first of the two adjacent amino acids with the non-side chain amino group of the second of the two adjacent amino acids;
wherein * between two adjacent amino acids indicates that the two adjacent amino acids are covalently connected by an isopeptide bond connecting the non-side chain carboxyl group of the first of the two adjacent amino acids with a side chain amino group of the second of the two adjacent amino acids.

Preferably, in the context of the invention, the term "amino acid" refers to an α-amino acid. Such an α-amino acid has an amino group attached to the α-carbon atom of a carboxyl group, therefore the α-carbon atom is attached to the carboxyl group. In addition to the carboxyl group and the amino group, to said α-carbon atom are additionally attached i) two hydrogen atoms, or ii) a hydrogen atom and a respective side chain, depending on the amino acid. For example, in the case of 2,3-Diaminopropionic acid the side chain is -(CH₂)-(NH₂). In the context of the invention, the amino group attached to the α-carbon atom and that is not part of the respective side chain is sometimes referred to as "non-side chain amino group". The carboxyl group to which the α-carbon atom is attached to and that is not part of the respective side chain is sometimes referred to as "non-side chain carboxyl group". When referring to the amino acid N Methyl-L-valine, the non-side chain amino group of *N*-Methyl-L-valine is a modified amino group, wherein one of the hydrogen atoms of the amino group of *N*-Methyl-L-valine is substituted with a methyl group.

In this context, the terminology "chain of amino acids" indicates, that sequential amino acids are connected by a carboxamide bond. Preferably, the carboxamide bond between each of the adjacent amino acids is independently selected from a peptide bond or an isopeptide bond.

However, this does not prerequisite that the amino acids X₁₅ and X₃, which are covalently connected, need to be connected by a carboxamide bond. Preferably, the amino acids X₁₅ and X₃, are covalently connected via ester bond.

In the context of the invention, the terms "carboxamide bond" and "carboxamide group" may be used interchangeably. Amongst others, a carboxamide bond can refer to a peptide bond or an isopeptide bond.

Preferably, in the context of the invention, a "peptide bond" refers to a carboxamide bond between a first and a second amino acid, wherein the carboxamide bond is between the non-side chain amino group of the first amino acid and the non-side chain carboxyl group of the second amino acid.

Preferably, in the context of the invention, an "isopeptide bond" refers to a carboxamide bond between a first and a second amino acid, wherein the carboxamide bond is between an amino group of the first amino acid and a carboxyl group of the second amino acid, wherein the amino group is a side chain amino group of the first amino acid and/or the carboxyl group is a side chain carboxyl group of the second amino acid.

Preferably, in the context of the invention, the term "L-Dap" refers to L-2,3-Diaminopropionic acid. Preferably, in the context of the invention, the term "*N*-methyl-L-Val" refers to N Methyl-L-valine. Preferably, in the context of the invention, the term *"D-allo-*Thr" refers to D-*Allo*-threonine. Preferably, in the context of the invention, the term "D-Ala" refers to D-Alanine. Preferably, in the context of the invention, the term "β-OH-L-*allo*-Asp" refers to (2S,3*S*)-3-hydroxyaspartic acid. Preferably, in the context of the invention, the term "β-OH-L-Phe" refers to (2*S*,3*R*)-3-hydroxyphenylalanine. Preferably, in the context of the invention, the term "D-*allo*-Ile" refers to D-*allo*-isoleucine . Preferably, in the context of the invention, the term "L-Ser" refers to L-Serine. Preferably, in the context of the invention, the term "D-Lys" refers to D-Lysine. Preferably, in the context of the invention, the term "D-val" refers to D-Valine. Preferably, in the context of the invention, the term "D-Leu" refers to D-Leucine. Preferably, in the context of the invention, the term "L-Leu" refers to L-Leucine. Preferably, in the context of the invention, the term "β-OH-L-Ile" refers to (2*S*,3*R*)-3-hydroxyisoleucine.

In preferred embodiments of the invention, the fatty acid group R is selected from a compound with the structure (I) 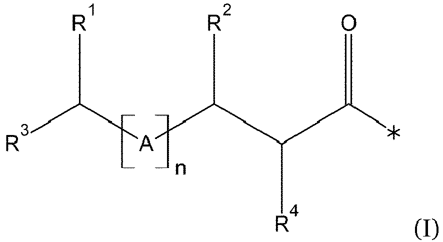
wherein each R¹, R³, and R⁴ are independently selected from the group consisting of: -H, -C₁₋₆alkyl, -C₂₋₆ alkenyl, -C₂₋₆ alkynyl and 3 to 6 membered cycloalkyl, wherein each H may independently be substituted by Cl and/or F, preferably wherein each R¹ is independently selected from the group -H, -CH₃, *tert*-butyl, isobutyl and cyclopropyl;
wherein R² is selected from the group consisting of -H, -CN, azido, -NO₂, -NH₂, -OH, -OCH₃₋ₓFₓ, - SH, -SCH₃₋ₓFₓ, -CH₃₋ₓFₓ (wherein x is 0 to 3), 3 to 6 membered cycloalkyl, 3 to 6 membered heterocyclyl, 3 to 6 membered aryl, and 3 to 6 membered heteroaryl, wherein each hydrogen atom in the 3 to 6 membered cycloalkyl, 3 to 6 membered heterocyclyl, 3 to 6 membered aryl or 3 to 6 membered heteroaryl may independently be substituted by a substituent selected from the group of -CN, halogen, (preferably wherein the halogen is F and/or Cl), azido, -NO₂, -NH₂, -OH, -OCH₃₋ₓFₓ, -SH, -SCH₃₋ₓFₓ, and -CH₃₋ₓFₓ, wherein x is 0 to 3, preferably wherein the heteroaryl or heterocyclyl contains at least one heteroatom selected from the group of nitrogen, oxygen, and/or sulphur;
wherein each of the A is independently selected from the group of -(CH₂)-, -(CH=CH)-, -(C=C)-, -(C=O)-, wherein each hydrogen atom of -(CH₂)- and -(CH=CH)- may independently be substituted with halogen, (preferably wherein the halogen is Cl, and/or F), and/or CH₃₋ₓFₓ, wherein x is 0 to 3; and
wherein n is 0 to 20.

In the above context, the term "cycloalkyl" or heterocyclyl" refers to a non-aromatic cyclic group. In the above context, the term aryl or heteroaryl refers to an aromatic cyclic group.

More preferably, n is 3 to 15, more preferably 5 to 10, more preferably 3 to 7, most preferably 5.

In preferred embodiments, each R¹, R³, and R⁴ are independently selected from the group of -H, -C₁₋₃ alkyl, -C₂₋₃ alkenyl, -C₂₋₃ alkynyl and 3 to 6 membered cycloalkyl, wherein each H may independently be substituted by Cl and/or F. In preferred embodiments of the invention, each R₁, R³, and R⁴ are independently selected from the group of -H, -C₁₋₃ alkyl, -C₂₋₃ alkenyl, and -C₂₋₃ alkynyl and 3 to 6 membered cycloalkyl. In preferred embodiments of the invention each R¹, R³, and R⁴ are independently selected from -CH₃ or -H. In preferred embodiments of the invention, R¹ is selected from -CH₃ or -H. In preferred embodiments of the invention, R³ is selected from - CH₃ or -H. In preferred embodiments of the invention, R⁴ is selected from -CH₃ or -H.

In preferred embodiments, R₂ is selected from the group consisting of -H, -CN, azido, -NO₂, -NH₂, -OH, -OCH₃₋ₓFₓ, -SH, -SCH₃₋ₓFₓ, -CH₃₋ₓFₓ, wherein x is 0 to 3. In preferred embodiments of the invention R² is -NH₂ or -OH.

In preferred embodiments, each of the A is independently selected from the group of -(CH₂)- and -(CH=CH)-, wherein each hydrogen atom of -(CH₂)- and -(CH=CH)- may independently be substituted with halogen, (preferably wherein the halogen is F), and/or CH₃₋ₓFₓ, wherein x is 0 to 3. In preferred embodiments of the invention A is -(CH₂)-.

In preferred embodiments of the invention,
n is 5;
R¹, R³, and R⁴ are each independently selected from -CH₃ or -H;
R² is -NH₂ or -OH; and
A is -(CH₂)-.

In preferred embodiments of the invention the fatty acid group R is selected from a compound with the structure 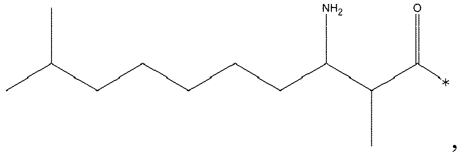 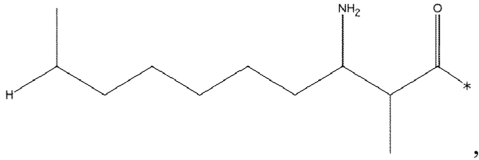 or

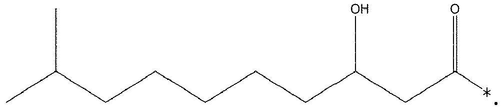

In this context, the star symbol in the above structure of the fatty acid groups R indicates that the fatty acid group R is bound to the chain of amino acids by the bond marked with the star symbol. In preferred embodiments of the invention the fatty acid group R is bound to the chain of amino acids via a carboxamide bond.

In preferred embodiments of the invention, the fatty acid group R is bound to the amino acid of the chain of amino acids at the X position with the lowest integer. Therefore, in these embodiments, the fatty acid group is
a) bound to the amino acid at the position X₁, or
b) in the case that the amino acid at the position X₁ is absent, the fatty acid group is bound to the amino acid at the position X₂.

In preferred embodiments, the chain of amino acids is chain 1

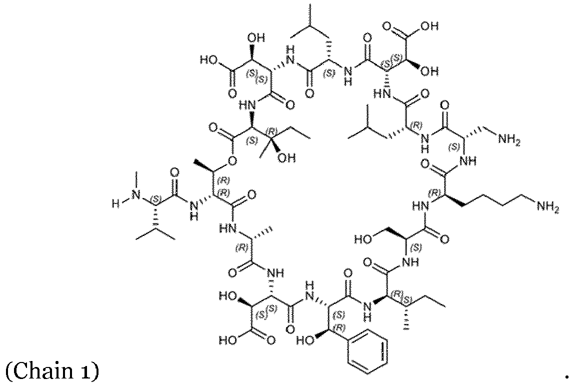

In preferred embodiments, the chain of amino acids is chain 2

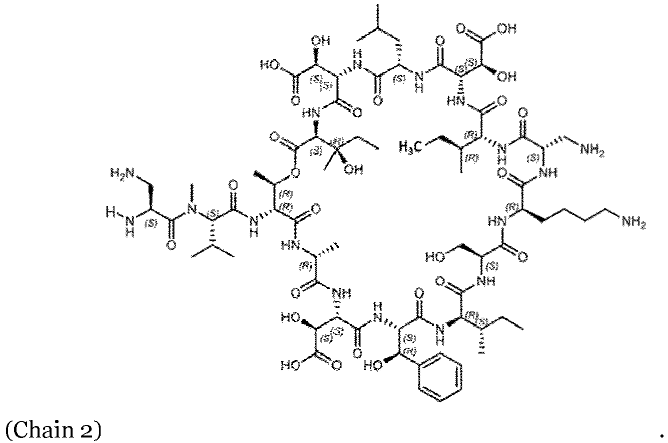

In preferred embodiments, the chain of amino acids is chain 3

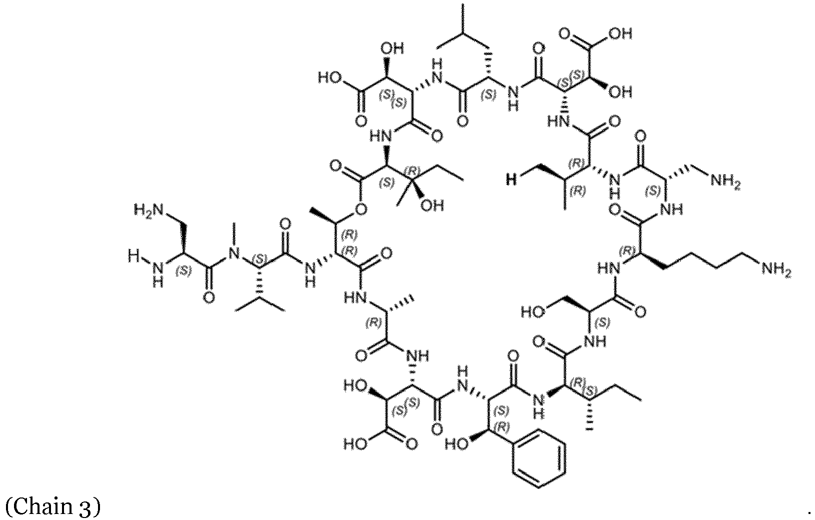

In preferred embodiments, the chain of amino acids is chain 4

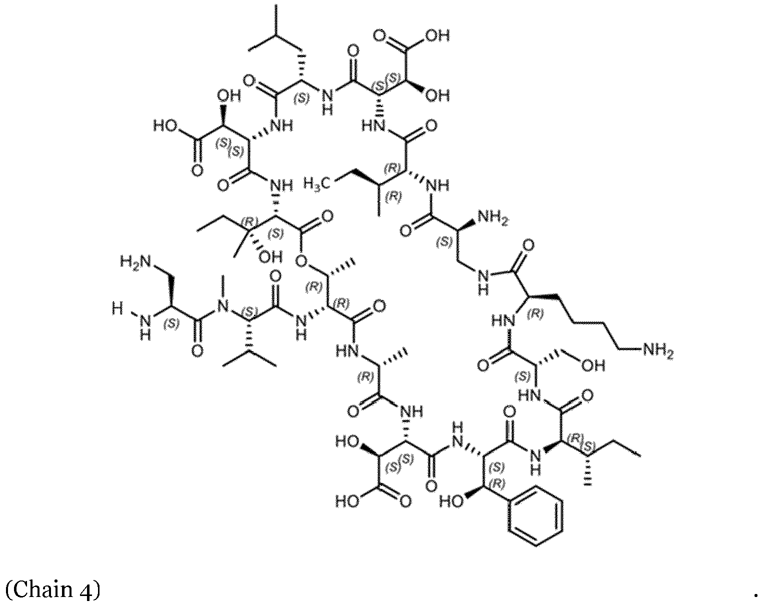

In preferred embodiments, the chain of amino acids is chain 5

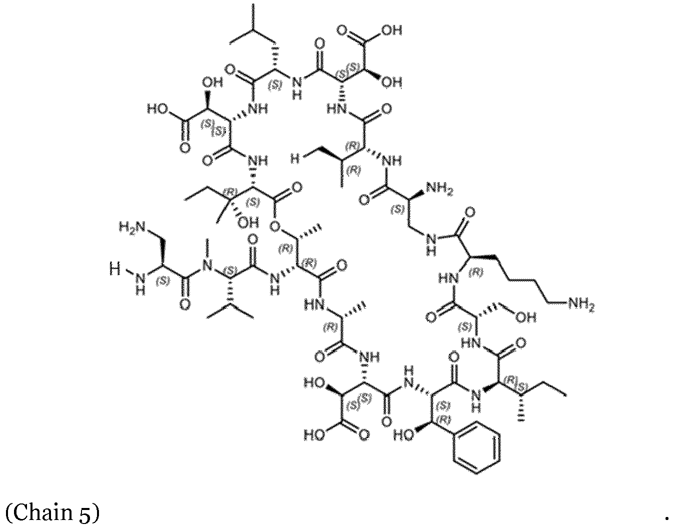

In preferred embodiments of the invention, the peptide is any one of the molecules with the structure (II-VII)
(II) **(SEQ ID NO. 6)** 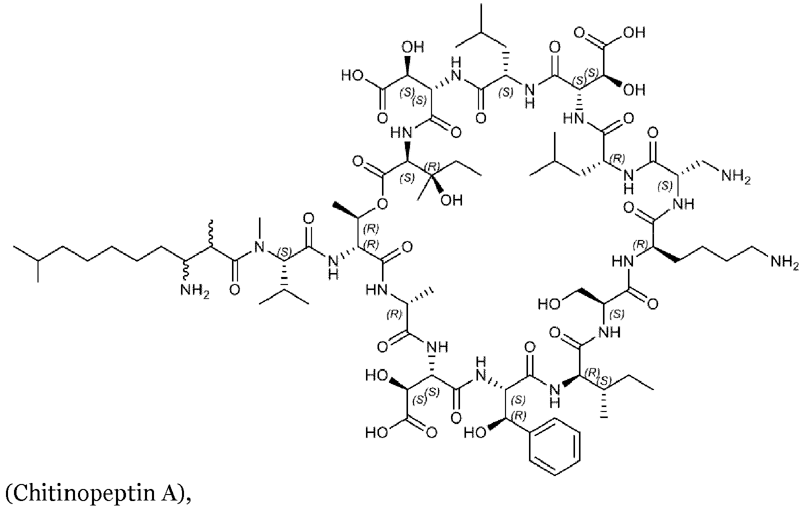
(III) **(SEQ ID NO. 6)** 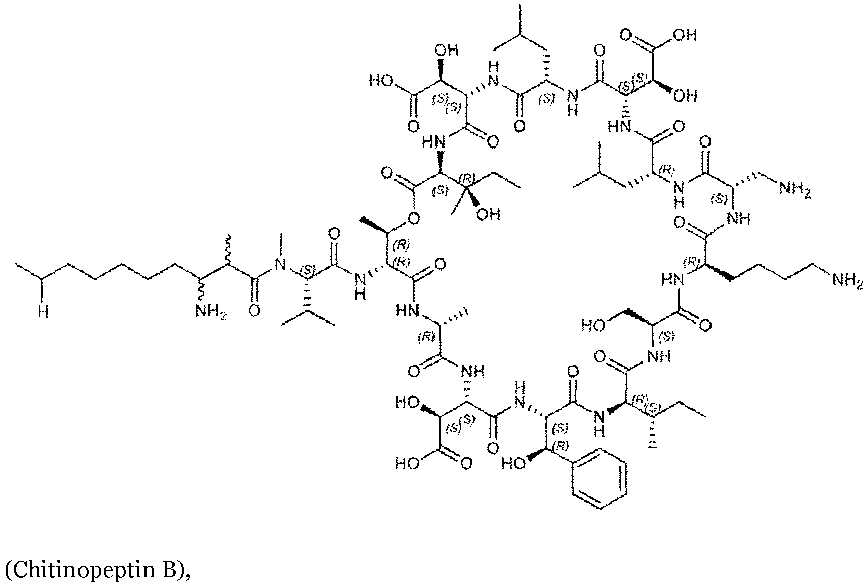
(IV) **(SEQ ID NO. 7**) 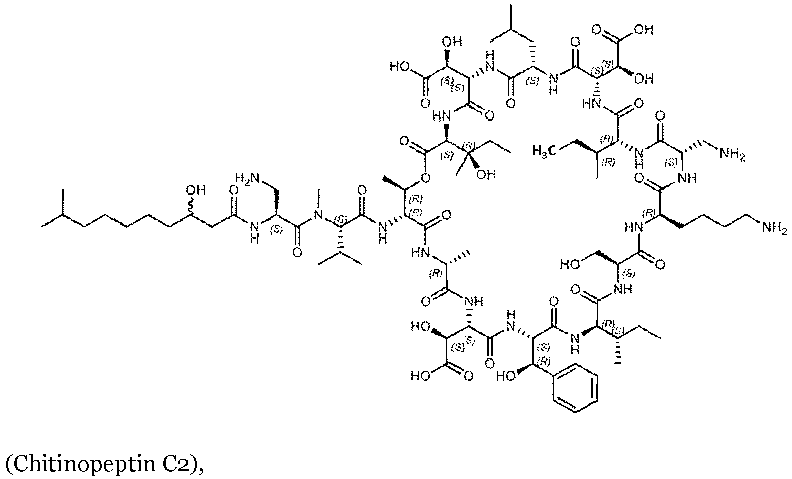
(V) **(SEQ ID NO. 8)** 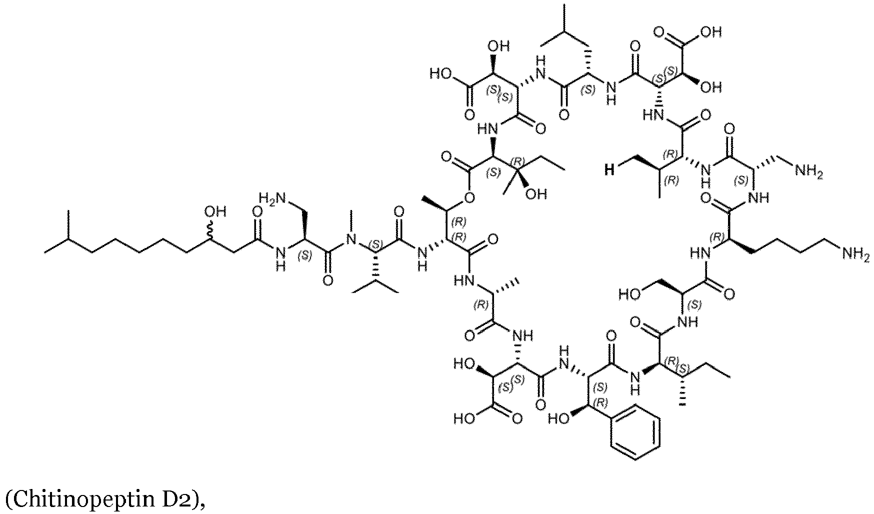
(VI) **(SEQ ID NO. 9 and 10**) 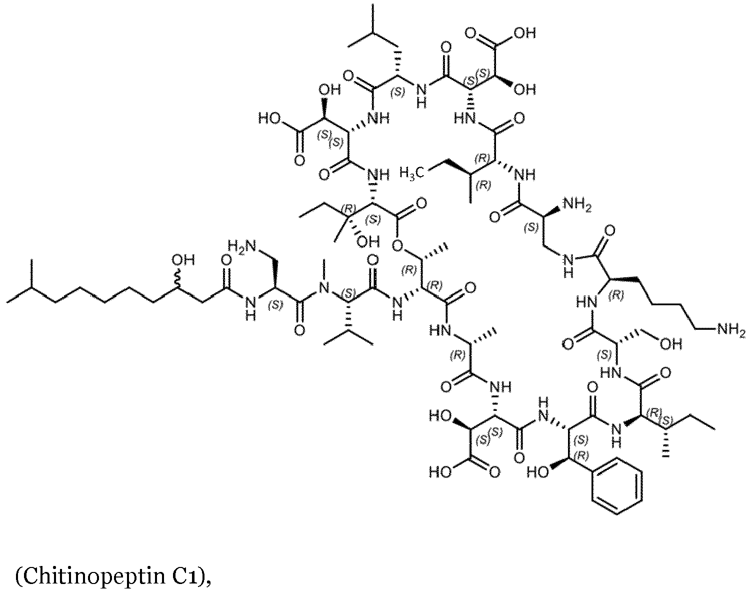
(VII) **(SEQ ID NO. 11 and 12**)

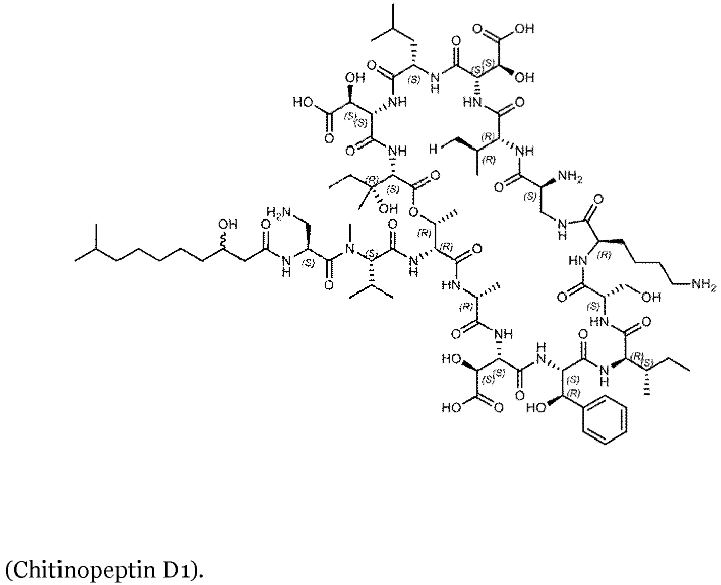

Preferably, the "wavy" bond in the above structures (II)-(VII) represents for the group marked with the "wavy" bond either each of the stereoisomers or a mixture of the stereoisomers.

**In** a **second aspect,** the invention pertains to a complex consisting of a peptide according any of the other aspects of the invention and a metal atom.

In this context, the peptide acts as a complexing agent that coordinates the metal atom. Without wishing to be bound by theory, the term metal atom may refer to any atom of an element which is commonly categorized as "metal" in the periodic table of elements. Preferably, the metal atom is a transition metal. Preferably, the metal is a single metal atom. For example, "metal atom" may refer to an atom, which is selected of the group of Li, Be, Na, Mg, Al, K, Ca, Sc, Ti, V, Cr, Mn, Fe, Co, Ni, Cu, Zn, Ga, Rb, Sr, Y, Zr, Nb, Mo, Tc, Ru, Rh, Pd, Ag, Cd, In, Sn, Cs, Ba, La, Ce, Pr, Nd, Pm, Sm, Eu, Gd, Tb, Dy, Ho, Er, Tm, Yb, Lu, Hf, Ta, W, Re, Os, Ir, Pt, Au, Hg, Tl, Pb, Bi, and Po. In preferred embodiments of the invention, the metal atom is selected from the group of Ru, Cu, Fe, Co, Re, Ag, Pt, Zn, Pd, Ir, Mn, Sn, V, Eu, Rh, Au, Ni, Hg, Bi, Gd, Ga, Cd, W, Yb, Lu, Cr, Er, Ta and Tl.

In further preferred embodiments of the invention, the metal atom is selected from the group of Fe, Cu, and Zn, preferably Fe. Without wishing to be bound by theory, the term "metal atom" does not indicate a specific oxidation state or a specific number of charges of the metal atom if not indicated otherwise. In preferred embodiments of the invention, the metal has three positive charges, such as in Fe³⁺.

**In a third aspect,** the invention pertains to a nucleic acid sequence encoding for the peptide according to any of the aspects of the invention.

The terms "polynucleotide", "oligonucleotide," and "nucleic acid sequence" are used interchangeably in the context of the present invention. These terms are used only to refer to the primary structure of the corresponding nucleic acid molecule. They preferably refer to polymeric (meaning two or more monomers) deoxyribonucleotides (containing 2-deoxy-D-ribose) or ribonucleotides (containing D-ribose) of any length. Preferably, the term "nucleic acid sequence" refers to polymeric deoxyribonucleotides, polyribonucleotides, as well as any other type of polymeric nucleotide (of any length) which is an N- or C-glycoside of a purine or pyrimidine base.

Preferably, the term "nucleic acid sequence" includes other polymers containing nonnucleotidic backbones, for example, polyamide (e.g., peptide nucleic acids (PNAs) such as PNAs comprising aminoethyl glycine units), polymorpholino, and other synthetic sequence-specific nucleic acid polymers providing that the polymers contain nucleobases in a configuration which allows for base pairing and base stacking, such as is found in DNA and RNA.

Preferably, the terms "nucleotide" and "polynucleotide" include 3'-deoxy-2',5'-DNA, oligodeoxyribonucleotide N3'→P5' phosphoramidates, 2'-O-alkyl-substituted RNA, double- and single-stranded DNA, as well as double- and single-stranded RNA, DNA: RNA hybrids, and hybrids between PNAs and DNA or RNA.

Preferably, the term "nucleic acid sequence" includes nucleic acid sequences with modifications, such as labels, which are known in the art. Preferably, the label is a radioactive, fluorescent, or other kind of tag. Preferably, the label is methylation, the inclusion of a cap structure, a substitution of one or more of the naturally occurring nucleotides with an analog, a internucleotide modification, modifications such as, for example, with uncharged linkages (e.g. methyl phosphonates, phosphotriesters, phosphoramidates, carbamates, or the like), with negatively charged linkages (e.g. phosphorothioates, phosphorodithioates, etc.), and with positively charged linkages (e.g. aminoalklyphosphoramidates, aminoalkylphosphotriesters), those containing pendant moieties, such as, for example, proteins (including nucleases, toxins, antibodies, signal peptides, poly-L-lysine, or the like), modifications with intercalators (e.g., acridine, psoralen, or the like), modifications containing chelators (e.g., metals, radioactive metals, boron, oxidative metals, or the like), modifications containing alkylators, modifications with modified linkages (e.g. alpha anomeric nucleic acids or the like), as well as unmodified forms of the polynucleotide or oligonucleotide.

Preferably, in the context of the invention, the terms "nucleoside" and "nucleotide" include those moieties which contain not only the known purine and pyrimidine bases, but also other heterocyclic bases which may have been modified. Preferably, such modifications include methylated purines or pyrimidines, acylated purines or pyrimidines, or other heterocycles. Preferably, modified nucleosides or nucleotides include modifications on the sugar moiety, e.g., wherein one or more of the hydroxyl groups are replaced with a halogen, an aliphatic group, or are functionalized as ethers, amines, or the like. Other preferred modifications to nucleotides or polynucleotides involve rearranging, appending, substituting for, or otherwise altering functional groups on the purine or pyrimidine base which form hydrogen bonds to a respective complementary pyrimidine or purine, e.g. isoguanine, isocysteine, and the like. In some embodiments, the oligonucleotides and/or probes include at least one, two, three or four modified nucleotides.

In some embodiments, the "nucleic acid sequence" comprises one or more universal bases. In the context of the invention, the term "universal base" refers to a nucleotide analog that can hybridize to more than one nucleotide selected from A, T, C, and G. Preferably, the universal base is selected from the group consisting of deoxyinosine, 3-nitropyrrole, 4-nitroindole, 6-nitroindole, 5-nitroindole.

In the context of the invention, a nucleic acid sequence encoding for the peptide according to any of the aspects of the invention refers to a nucleic acid sequence that, when expressed, results in a peptide sequence according to the invention. In preferred embodiments, said nucleic said sequence encoding for the peptide may not directly code for a peptide according to the invention but encode for a cellular machinery, for example a non-ribosomal peptide synthetase (NRPS), which is capable of synthesizing the peptide according to the invention. Preferably, the nucleic acid sequence encodes for the synthesis of the peptide according to the invention.

In preferred embodiments, the nucleic acid sequence encodes for a NRPS that is able to synthesize the peptide according to any aspect of the invention.

Preferably, the nucleic acid sequence that is encoding for the NRPS is a nucleic acid sequence according to any other aspect of the invention. Preferably, the nucleic acid sequence encoding the peptide or NPRS is contained in a plasmid.

**In a fourth aspect, the invention pertains to an NRPS** that is able to synthesise the peptide according to any one of the other aspects of the invention. Preferably, the NRPS is not able to completely synthesise the peptide according to the present invention.

Without wishing to be bound by theory, non-ribosomal peptide synthetases are enzymes found in many bacteria and fungi, and are known to catalyze the production of biologically active peptides from amino acid or related monomer precursors without the need for a nucleic acid template. In the context of the invention, the term "NRPS" refers to a biosynthetic enzyme that catalyzes the linkage of constituents to form a non-ribosomal peptide, preferably wherein the constituent is an amino acid.

Without wishing to be bound by theory, peptides are synthesized by NRPS in a modular assembly line-like manner by NRPS enzymes. NRPS comprise sets of modules, each of which consists of various functional domains such as adenylation (A), condensation (C), cyclization (Cy), thiolation (T, also known as peptidyl carrier domain (PCP)), methyltransferase (Mt), epimerization (E) or thioesterase (TE) domains. The modules that comprise an NRPS template may be clustered on a single enzyme or located within multiple distinct enzymes that associate post-translation; and are classified as either initiation, termination, or elongation modules depending on their location in the assembly line. Within each module of an NRPS, an adenylation (A) domain recognizes and activates a specific substrate by addition of AMP. The activated substrate is then tethered to a flexible 4'-phosphopantetheine (PPT) prosthetic group, which is itself covalently attached to a thiolation (T) domain (also known as a peptidyl carrier protein (PCP) domain). Post-attachment of an activated substrate by its A domain partner, a T domain then transfers that substrate to a condensation (C) domain, which catalyses peptide bond formation between the donor substrate provided by the T domain immediately upstream, and the acceptor substrate provided by the downstream T domain. Following the initial condensation event, the process can repeat in an iterative fashion, with the previous peptide intermediate now serving as the donor substrate for the C domain of the next module in an NRPS complex. Along the way, certain modules may contain additional tailoring domains that modify individual substrates in a directed fashion (e.g., epimerisation (E) domains, for conversion from L- to D-enantiomers or methyltransferase (Mt) domains for attaching a Methyl to the substrate). In this process, the growing peptide continues to be passed from the T domain of one module to the T domain of the next until the product is released, typically via a hydrolysis or intramolecular cyclisation reaction catalysed by one or two thioesterase (TE) domain(s) associated with the final module in the NRPS complex.

In the context of the invention, the expressions "NRPS that is able to synthesise the peptide" or "the peptide is synthesized by the NRPS" do not stipulate that the NPRS needs to be the only enzyme involved in the synthesis of the peptide. In such a case, it is preferred that the NRPS is one of several enzymes that are involved in the synthesis of the peptide. Such enzymes are thioesterase and diiron-monooxygenase and optionally enzymes for the precursor supply of 2,3-diaminopropionic acid (Dap). Preferably, the thioesterase and diiron-monooxygenase catalyse substrate hydroxylation. Preferably, the NRPS synthesizes the chain of amino acids and optionally attaches the fatty acid group to the chain of the amino acids.

**In a fifth aspect,** the invention pertains to a vector, comprising a nucleic acid sequence according to any other aspect of the invention. Preferably, the vector comprises a nucleic acid sequence according to the third aspect of the invention.

A vector in the context of the invention refers to a protein, nucleic acid or mixture thereof which is capable of being introduced or of introducing the nucleic acid sequence, such as the nucleic acid sequence encoding for the NRPS or a nucleic acid sequence encoding for the peptide, into a cell. Preferably, the proteins and/or the peptides encoded by the introduced nucleic acid sequence are expressed within the cell upon introduction of the vector.

In preferred embodiments of the invention the vector comprises recombinant vectors, plasmids, phagemids, phages, artificial chromosome or minichromosomes, transposons, cosmids, fosmids, viruses, virosomes, and nucleic acid coated particles, in particular gold spheres.

The vector may further comprise an expression regulatory sequence (for example, an enhancer sequence, a Kozak sequence, a polyadenylation sequence, a transcriptional termination sequence, etc.), a selection marker (for example, a drug resistance factor) sequence, an origin of replication, and the like.

Preferably, the vector according to the present invention is a recombinant vector. Preferably, the recombinant vector is an expression vector, optionally comprising one or more genes to be expressed. Preferably, said expression is driven by a regulatory sequence (or sequences). A regulatory sequence can be isolated from a naturally occurring genomic DNA sequence or can be synthetic, for example, a synthetic promoter.

A preferred regulatory sequence useful for the expression of a nucleotide sequence according to the invention is a promoter element. In preferred embodiments, promoters include, but are not limited to, constitutive, inducible, temporally regulated, developmentally regulated, spatially regulated, chemically regulated, stress-responsive, tissue-specific, viral and synthetic promoters. Promoter sequences are known to be strong or weak. A strong promoter provides for a high level of gene expression, whereas a weak promoter provides for a very low level of gene expression. An inducible promoter is a promoter that provides for the turning on and off of gene expression in response to an exogenously added agent, or to an environmental or developmental stimulus. For example, a bacterial promoter can be induced to varying levels of gene expression depending on the level of isopropyl-thiogalactoside added to transformed bacterial cells. An isolated promoter sequence that is a strong promoter for heterologous nucleic acids is advantageous because it provides for a sufficient level of gene expression to allow for easy detection and selection of transformed cells and provides for a high level of gene expression when desired.

The nucleic acid sequence may be a recombinant nucleic acid molecule. The vector may be a recombinant vector. The term "recombinant nucleic acid molecule" or "recombinant vector" refers to a nucleic acid sequence or to a vector produced by human intervention. A recombinant nucleic acid molecule can contain two or more nucleotide sequences that are linked in a manner such that the product is not found in a cell in nature. In particular, the two or more nucleic acid sequences can be operatively linked and, for example, can encode a fusion polypeptide, or can comprise a nucleotide sequence and a regulatory element.

A recombinant nucleic acid molecule also can be based on, but different, from a naturally occurring nucleic acid sequence, for example, a polynucleotide having one or more nucleotide changes such that a first codon, which normally is found in the polynucleotide, is replaced with a degenerate codon that encodes the same or a conservative amino acid, or such that a sequence of interest is introduced into the polynucleotide, for example, a restriction endonuclease recognition site or a splice site, a promoter, a DNA replication initiation site, or the like.

**In a sixth aspect,** the invention pertains to a cell comprising the peptide, the complex, the nucleic acid sequence, the NRPS, and/or the vector according to any one of the other aspects of the invention.

**In a seventh aspect,** the invention pertains to a plant protection agent comprising the peptide, the complex, a nucleic acid sequence, the NRPS, the vector and/or the cell according to any one of the other aspects of the invention.

Preferably, the plant protection agent is in the form of a liquid, dust, granules, microgranules, pellets, wettable powder, flowable powder, emulsions, microcapsules, oils, or aerosols. Preferably the plant protection agent is packaged and/or sold in a concentrated form that is diluted prior to application.

In preferred embodiments of the invention, the plant protection agent further comprises a source of nutrients such as a source of carbon, nitrogen, vitamins, micronutrients and amino acids. Preferably, the nutrients enhance the growth of the plant and/or are beneficial to the synthesis of the peptide and/or complex according to the invention.

Preferably, the plant protection agent comprises an adjuvant. An adjuvant in this context refers to a compound or preparation, which enhances the efficacy of active ingredients (such as the peptide), and preferably improves the overall performance of the plant protection agent. The adjuvant may act, for example, by increasing the wetting or the adhesion of the plant protection agent, or by prevention of foaming. Preferably, said adjuvant is selected from the group of wetting agents, adhesion agents and/or anti-foaming agents.

**In an eighth aspect,** the invention pertains to a method of manufacturing a peptide, the complex, the NRPS and/or the plant protection agent according to any aspect of the invention, wherein the method comprises a recombinant and/or a chemical synthesis.

In preferred embodiments, the method of manufacturing is conducted in an organism, preferably wherein the organism is a bacterium (such as *Escherichia coli, Pseudomonas* sp., *Myxococcus* sp., *Bacillus* sp., or *Streptomyces* sp.) and/or a fungus (such as *Aspergillus* sp., *Fusarium* sp., or *Saccharomyces* sp.). In preferred embodiments of the invention, the method of manufacturing is conducted in a cell, preferably in a fungus cell and/or a bacterial cell. In preferred embodiments of the invention, the organism comprises the cell.

In preferred embodiments, the method of manufacturing comprises culturing the organism and/or cell in a culture broth. Preferably, after culturing the cell and/or organism in the culture broth, the peptide and/or the complex is separated from the culture broth. Preferably, the separation of the peptide and/or the complex from the culture broth is conducted by solvent extraction. Preferably, the extraction solvent is a polar extraction solvent. Preferably, the polar extraction solvent is selected from benzyl alcohol, 1-propanol, acetic acid, 2-aminoethanol, ethanol, diethylene glycol, methanol, ethylene glycol, glycerine, and/or water.

In preferred embodiments of the invention, the fungus is a yeast. In preferred embodiments of the invention, the fungus cell is a yeast cell.

Preferably, the cell is a host cell. "Host cell" as used in this context refers to a cell, into which the nucleic acid sequence and/or the vector that has been introduced (e.g. transformed, transduced, infected or transfected). Host cells of the present invention may include bacterial cells and/or fungus cells. Preferably, the host cell a cell according to the sixth aspect of the invention.

In preferred embodiments of the invention, the method comprises introducing, preferably transducing, transforming or transfecting, the cell, preferably the bacterial and/or fungus cell, with a vector according to a previous aspect of the invention.

Such modified host cells of the present invention preferably refer to host cells, wherein the nucleic acid sequence is operably linked to a promoter.

In preferred embodiments of the invention, the organism is a bacterium, preferably a bacterium of the phylum *Bacteroidetes,* preferably of the class of *Chitinophagia,* preferably of the genus *Chitinophaga.* In preferred embodiments of the invention, said bacterium is *C. eiseniae* or *C. flava, C. niastensis* and/or *C. oryziterrae.* Preferably, the abovementioned organisms are a host cell according to the invention.

In preferred embodiments, the method comprises an expression, preferably a heterologous expression, of a NRPS. In preferred embodiments, the peptide is synthesized by the NRPS. Preferably, the NRPS is an NRPS according to another aspect of the invention. Preferably, the peptide is partially synthesized by the NRPS. For example, the NRPS may synthesize a precursor of the peptide.

In preferred embodiments, the method of manufacturing comprises the sequential steps:
(i) culturing the cell and/or the organism in a culture broth;
(ii) optionally, freeze-drying the culture broth;
(iii) separating the peptide and/or the complex from the culture, preferably by solventextraction;
(iv) optionally, purifying the peptide and/or the complex, for example by using a chromatography-based method.

**In a ninth aspect, the invention** pertains to a plant protection agent, obtainable by the method according to any one of the other aspects of the invention. Preferably, the plant protection agent according to any aspect of the invention is an extract, a purified compound or mixture of several purified compounds.

**In a tenth aspect,** the invention pertains to a method for treating a disease and/or increasing resistance against an infection in a plant, wherein method comprises administering to the plant a peptide, complex and/or plant protection agent according to any one of the other aspects of the invention.

In the context of the invention, "treatment" or "treating" refers to the eradicating, reducing, ameliorating, reversing, or preventing of a degree, sign or symptom of a condition or disease to any extent, and includes, but does not require, a complete cure of the condition or disease. Treating can be curing, improving, or partially ameliorating a disorder. In some embodiments, treatment can comprise controlling a pathogen that causes an infection, infestation, or other disease. In the context of the invention the term "control" of a pathogen or an infection therewith extends to the act of killing, disabling, immobilizing, or reducing population numbers of a pathogen and/or rendering the pathogen substantially incapable of causing harm. For example, the term "pesticidal" means capable of controlling a pathogen, and "fungicidal" means capable of controlling a fungal pathogen.

In preferred embodiments of the invention, the disease and/or infection is a fungal and/or bacterial disease and/or infection. Preferably, the disease is a bacterial infection. In the context of the invention, the fungal and/or bacterial disease and/or infection is associated with the presence of a bacterial or fungal pathogen respectively. In preferred embodiments of the invention, wherein the bacterial infection is an infection with a Gram-negative bacterium. In preferred embodiments of the invention, the Gram-negative bacterium is of the genus *Erwinia, Pseudomonas, Ralstonia, Burkholderia, Xanthomonas,* and *Agrobacterium* or *Xylella.* In preferred embodiments of the invention, the bacterial infection is an infection with a Gram-positive bacterium. Preferably, the Gram-positive bacterium is of the generum *Clavibacter, Curtobacterium, Arthrobacter Bacillus, Rhodococcus, Clostridium* or *Streptomyces.* In preferred embodiments of the invention, the bacterial infection is an infection with a *Xylella* bacterium, preferably, wherein the *Xylella* bacterium is *Xylella fastidiosa.*

In certain embodiments, the methods and compositions according to the subject invention reduce damage to a plant caused by a pathogen, preferably the bacterium, preferably *Xylella fastidiosa,* by about 5%, 10%, 20%, 30%, 40%, 50%, 60% 70%, 80%, or 90% or more, compared to a plant that was not treated according to the method of the present invention. In certain embodiments, the methods and compositions according to the subject invention lead to an increase in crop yield by about 5%, 10%, 20%, 30%, 40%, 50%, 60% 70%, 80%, or 90% or more, compared to untreated crops. In one embodiment, the methods of the subject invention lead to a reduction in the amount of *Xylella fastidiosa* in or on a plant or in a plant's surrounding environment by about 5%, 10%, 20%, 30%, 40%, 50%, 60% 70%, 80%, or 90% or more, compared to a plant that was not treated according to the method of the present invention. In one embodiment, the methods of the subject invention lead to an increase in the mass of a plant and/or a plant's fruit by about 5%, 10%, 20%, 30%, 40%, 50%, 60% 70%, 80%, or 90% or more, compared to a plant that was not treated according to the method of the present invention.

In preferred embodiments of the invention, the plant is susceptible to a bacterial infection. Without wishing to be bound by theory, a susceptible plant preferably refers to a plant that is at risk of becoming infected. Preferably such a susceptible plant is not or only to a limited degree able to restrict the growth and development of a specified pathogen. In this context, a plant that is at higher risk of becoming infected and/or is worse at being able to restrict the growth and development of a specified pathogen is more susceptible. A plant that is at lower risk of becoming infected and/or is better at being able to restrict the growth and development of a specified pathogen is less susceptible. In this context of the invention, "increasing resistance" has the same effect as "lowering susceptibility".

Preferably, the plant is selected from the group of *Adoxaceae* (e.g. *Sambucus, Viburnum),* Altingiaceae (e.g. *Liquidambar), Amaranthaceae* (e.g. *Alternanthera*, *Amaranthus, Atriplex, Chenopodiastrum, Chenopodium, Dysphania, Kali), Anacardiaceae* (e.g. *Rhus, Toxicodendron, Pistacia), Apiaceae* (e.g. *Conium, Coriandrum, Datura, Daucus, Oenanthe), Apocynaceae* (e.g. *Catharanthus, Nerium, Vinca), Arecaceae (Phoenix), Arialiaceae* (*Meryta*), *Aquifoliales* (such as *Ilex) Araliaceae* (e.g. *Fatsia, Hedera), Araucariaceae* (e.g. *Agathis*), *Argophyllaceae (e.g. Corokia), Asparagaceae* (e.g. *Asparagus, Cordyline), Asphodelaceae (e.g. Hemerocallis, Phormium), Asteraceae* (e.g. *Ambrosia, Argyranthemum, Artemisia, Baccharis, Brachyglottis, Bidens, Callistephus, Calyptocarpus, Chrystanthemum, Conyza, Dimorphotheca, Encelia, Erigeron, Eriocephalus, Euryops, Facelis, Franseria, Helianthus, Helichrysum, Heterotheca, Hypochaeris, Iva, Lactuca, Osteospermum, Phagnalon, Pluchea, Ratibida, Santolina, Senecio, Silybum, Solidago, Sonchus, Symphyotrichum, Taraxacum, Vernonia, Xanthium*), *Athyriaceae* (e.g. *Athyrium*), *Betulaceae* (e.g. *Alnus*), *Bignoniaceae* (e.g. *Chitalpa, Campsis, Jacaranda), Boraginaceae* (e.g. *Amsinckia, Echium, Heliotropium), Brassicaceae* (e.g. *Arabidopsis, Brassica, Capsella, Erysimum, Lepidium, Lobularia, Sisymbrium), Cannabaceae* (e.g. *Celtis, Humulus), Cannaceae* (e.g. *Canna*), *Caprifoliaceae* (e.g. *Lonicera, Symphoricarpos), Celastraceae (e.g. Celastrus), Cistaceae (e.g. Cistus), Commelinaceae (e.g. Commelina), Caryophyllaceae (e.g. Stellaria), Cucurbitales (e.g. Diplocyclos), Convolvulaceae* (e.g. *Convolvulus, Ipomoea, Merremia macrocalyx), Cornaceae* (e.g. *Cornus), Corynocarpaceae (e.g. Corynocarpus) Cupressaceae (Juniperus), Cyperaceae* (e.g. *Carex, Cyperus), Dennstaedtiaceae (Pteridium), Ebenaceae* (e.g. *Diospyros), Elaeagnaceae (Elaeagnus) Ericaceae* (e.g. *Arbutus, Arctostaphylos, Calluna, Erica, Vaccinium), Escalloniaceae (Escallonia)* Euphorbiaceae (e.g. *Croton, Euphorbia, Hevea, Mallotus), Fabaceae* (e.g. *Acacia, Adenocarpus, Albizia, Anthyllis, Calico tome, Cercis, Chamaecrista, Coronilla, Cytisus, Dermatophyllum, Genista, Gleditsia, Lathyrus, Lupinus, Medicago, Melilotus, Mimosa, Neptunia, Retama, Robinia, Senna, Spartium, Swainsona, Trifolium, Ulex, Vicia, Wisteria), Fagaceae* (e.g. *Fagus, Quercus), Geraniaceae* (e.g. *Erodium, Geranium, Pelargonium), Gesneriaceae* (e.g. *Streptocarpus)* Haloragaceae (e.g. *Haloragis), Ginkgoaceae (Ginkgo), Hydrangeaceae* (e.g. *Hydrangea), Hypericaceae (e.g. Hypericum), Juglandaceae* (e.g. *Carya, Juglans), Lamiaceae* (e.g. *Callicarpa, Lavandula,* Ocimum, *Origanum, Perovskia, Phlomis, Marrubium, Melissa, Mentha, Rosmarinus, Salvia, Stachys, Teucrium, Westringia), Lauraceae* (e.g. *Laurus, Persea, Sassafras, Umbellularia*), *Lythraceae (Lagerstroemia) Magnoliaceae,* (e.g. , *Liriodendron, Magnolia), Malvaceae* (e.g. *Hibiscus, Malva, Modiola, Sida),* Moraceae, (e.g. *Broussonetia, Ficus, Morus), Montiaceae (e.g. Montiastrum) Myrtaceae* (e.g. *Callistemon, Eucalyptus, Eugenia, Leptospermum, Metrosideros, Myrtus, Psidium, Syzygium), Oleaceae* (e.g. *Chionanthus, Fraxinus, Ligustrum, Olea, Phillyrea, Syringa), Nyctaginaceae* (e.g. *Boerhavia), Onagraceae* (e.g. *Epilobium, Fuchsia, Clarkia, Ludwigia, Oenothera*), *Passifloraceae* (*e.g. Passiflora), Pinaceae* (*e.g. Pinus), Pittosporaceae* (e.g. *Pittosporum), Plantaginaceae* (e.g. *Hebe, Plantago), Platanaceae* (e.g. *Platanus), Poaceae* (e.g. *Agrostis, Anisantha, Avena, Axonopus, Brachiaria, Bromus, Cenchrus, Chloris, Coelorachis, Cynodon, Digitaria, Echinochloa, Eleusine, Eragrostis, Eriochloa, Eriochola, Festuca, Holcus, Hordeum, Lolium, Panicum, Paspalum, Pennisetum, Phalaris, Phleum, Poa, Setaria, Sorghum), Polygonaceae* (e.g. *Eriogonum, Fagopyrum, Fallopia, Persicaria, Polygonum, Rheum, Rumex*)*, Portulacaceae* (e.g. *Montia, Portulaca), Ranunculaceae* (e.g. *Clematis, Ranunculus), Resedaceae* (e.g. *Reseda), Rhamnaceae* (e.g. *Rhamnus), Rosaceae* (e.g. *Cotoneaster, Fragaria, Photinia, Pyrus, Prunus, Rosa, Rubus), Rubiaceae* (e.g. *Borreria, Coffea, Coprosma, Richardia), Rutaceae* (e.g. *Citrus, Melicope, Ruta), Polygalaceae (e.g. Polygala), Salicaceae* (e.g. *Populus, Salix), Sapindaceae* (e.g. *Acer, Aesculus, Alectryon, Dodonaea, Koelreuteria, Sapindus), Scrophulariaceae* (e.g. *Eremophila, Myoporum, Veronica), Simmondsiaceae* (e.g. *Simmondsia), Solanaceae* (e.g. *Datura, Lycopersicon, Nicotiana, Solanum), Strelitziaceae (e.g. Strelitzia),* Talinaceae (e.g. Talinum), Theaceae (e.g. *Stewartia) Ulmaceae* (e.g. *Ulmus) Urticaceae* (e.g. *Urtica), Proteaceae (e.g. Grevillea, Hakea), Verbenaceae* (e.g. *Duranta, Verbena), Violaceae* (e.g. *Melicytus)* and *Vitaceae* (e.g. *Ampelopsis, Parthenocissus*, *Vitis*).

In preferred embodiments of the invention, the plant is selected from the group of *Acacia* (such as *Acacia cultriformis*, *Acacia dealbata, Acacia longifolia, Acacia melanoxylon, Acacia saligna), Acer* (such *as Acer griseum, Acer macrophyllum, Acer negundo, Acer platanoides, Acer pseudoplatanus, Acer rubrum, Acer saccharum), Adenocarpus lainzii, Aesculus* (such as *Aesculus californica, Aesculus* × *hybrida), Agathis australis, Agrostis gigantea, Albizia julibrissin, Alectryon excelsus, Alnus rhombifolia, Alternanthera ficoidea, Amaranthus* (such as *Amaranthus blitoides, Amaranthus retroflexus), Ambrosia* (such as *Ambrosia acanthicarpa, Ambrosia artemisiifolia, Ambrosia psilostachya, Ambrosia trifida, Ambrosia trifida* var. *Texana), Ampelopsis* (such as *Ampelopsis arborea, Ampelopsis brevipedunculata, Ampelopsis brevipedunculata* var. *Hancei, Ampelopsis cordata), Amsinckia douglasiana, Anisantha* (such as *Anisantha diandra*, *Anisantha rigida), Anthyllis hermanniae, Arabidopsis thaliana, Arbutus unedo, Arctostaphylos, Argyranthemum frutescens, Artemisia* (such as *Artemisia absinthium, Artemisia arborescens, Artemisia douglasiana), Asparagus acutifolius, Athyrium filix-femina, Atriplex, Avena fatua, Axonopus compressus, Baccharis* (such as *Baccharis halimifolia, Baccharis pilularis, Baccharis salicifolia), Berberidaceae (Nandina), Bidens pilosa, Boerhavia diffusa, Borreria latifolia, Brachiaria* (such as *Brachiaria decumbens, Brachiaria plantaginea), Brachyglottis, Brassica* (such as *Brassica nigra), Bromus, Broussonetia papyrifera, Calicotome* (such as *Calicotome spinosa, Calicotome villosa), Callicarpa americana, Callistemon citrinus, Callistephus chinensis, Calluna vulgaris, Calyptocarpus biaristatus, Campsis radicans, Canna, Capsella bursa-pastoris, Carex, Carya* (such as *Carya aquatica, Carya cathayensis, Carya cordiformis*, *Carya floridana, Carya glabra, Carya illinoinensis, Carya laciniosa, Carya pallida, Carya palmeri, Carya, Carya tomentosa), Catharanthus* (such as *Catharanthus roseus), Celastrus orbiculatus, Celtis occidentalis, Cenchrus echinatus, Ceratochloa cathartica, Cercis* (such as *Cercis canadensis, Cercis occidentalis, Cercis siliquastrum), Chamaecrista fasciculata, Chenopodiastrum murale, Chenopodium* (such as *Chenopodium album, Chenopodium quinoa), Chionanthus* (such as *Chionanthus retusus), Chitalpa tashkentensis, Chloris halophila, Cistus* (such as *Cistus albidus, Cistus creticus, Cistus inflatus, Cistus monspeliensis, Cistus salviifolius, Cistus* × *incanus), Citroncirus webberi, Citrus* (such as *Citrus aurantiifolia, Citrus aurantium, Citrus celebica, Citrus clementina, Citrus clementina* × *C. sinensis, Citrus deliciosa* × C. *sinensis, Citrus jambhiri, Citrus limon, Citrus macrophylla, Citrus medica, Citrus natsudaidai, Citrus paradisi, Citrus reshni, Citrus reticulata, Citrus sinensis, Citrus sunki, Citrus tangerina, Citrus unshiu, Citrus* × *limonia, Citrus* × *nobilis, Citrus* × *tangelo), Clarkia amoena* subsp. *Lindleyi, Clematis cirrhosa, Coelorachis cylindrica, Coffea* (such as *Coffea arabica, Coffea arabica* × *C. canephora, Coffea arabica* × *C. eugenioides, Coffea arabica* × *C. liberica* var. *Dewevrei, Coffea arabica* × *C.racemosa, Coffea canephora, Coffea eugenioides, Coffea kapakata, Coffea liberica, Coffea liberica* var. *Dewevrei, Coffea racemosa, Coffea stenophylla), Commelina* (such as *Commelina benghalensis, Commelina erecta), Conium maculatum, Convovulvus* (such as *Convolvulus arvensis, Convolvulus cneorum), Coprosma* (such as *Coprosma baueri, Coprosma repens, Coprosma robusta), Cordyline* (such as *Cordyline australis), Coriandrum sativum, Cornusflorida, Corokia* (such as *Corokia cotoneaster, Corokia macrocarpa), Coronilla* (such as *Coronilla valentina, Coronilla valentina* subsp. *Glauca), Corynocarpus laevigatus, Cotoneaster rotundifolius, Croton setigerus, Cynodon dactylon, Cyperus* (such as *Cyperus eragrostis, Cyperus esculentus), Cytisus* (such as *Cytisus scoparius, Cytisus spinosa, Cytisus villosus), Datura wrightii, Daucus carota* subsp. *Sativus, Dendranthema* × *grandiflorum*, *Digitaria* (such as *Digitaria horizontalis, Digitaria insularis, Digitaria sanguinalis), Diospyros kaki, Diplocyclos palmatus, Dodonaea viscosa, Duranta erecta, Dysphania ambrosioides, Echinochloa crus-galli, Echium plantagineum, Elaeagnus* (such as *Elaeagnus angustifolia, Elaeagnus* × *submacrophylla), Eleusine indica, Encelia farinosa, Epilobium* (such as *Epilobium brachycarpum, Epilobium ciliatum), Eragrostis diffusa, Eremophila maculata, Erica cinerea, Erigeron* (such as *Erigeron bonariensis, Erigeron canadensis, Erigeron karvinskianus, Erigeron sumatrensis), Eriocephalus africanus, Eriochloa* (such as *Eriochloa contracta, Eriochloa gracilis), Eriogonum, Erodium* (such as *Erodium botrys, Erodium cicutarium, Erodium moschatum), Erysimum* hybrids, *Escallonia bifida, Eucalyptus* (such as *Eucalyptus camaldulensis, Eucalyptus globulus), Euphorbia* (such as *Euphorbia chamaesyce, Euphorbia hirta, Euphorbia terracina), Euryops* (such as *Euryops chrysanthemoides, Euryops pectinatus), Facelis retusa, Fagopyrum esculentum, Fagus crenata, Fallopia* (such as *Fallopia convolvulus, Fallopia japonica), Fatsia japonica, Ficus carica, Fragaria vesca* subsp. *Californica, Fraxinus* (such as *Frangula alnus, Fraxinus americana, Fraxinus angustifolia, Fraxinus dipetala, Fraxinus latifolia, Fraxinus pennsylvanica), Fuchsia magellanica, Genista* (such as *Genista corsica, Genista ephedroides, Genista hirsuta, Genista lucida, Genista scorpius, Genista tridentata, Genista valdes-bermejoi, Genista* × *spachiana), Geranium dissectum, Ginkgo biloba, Gleditsia* (such as *Gleditsia triacanthos, Gleditsia triacanthos* var. *inermis), Grevillea* (such as *Grevillea alpina, Grevillea juniperina), Hakea petiolaris, Haloragis erecta, Hebe* (such as *Hebe elliptica), Hedera helix, Helianthus* (such as *Helianthus annuus), Helichrysum* (such as *Helichrysum italicum, Helichrysum stoechas), Heliotropium* (such as *Heliotropium europaeum, Heliotropium fruticosum, Heliotropium indicum), Hemerocallis, Heterotheca grandiflora, Hevea brasiliensis, Hibiscus* (such as *Hibiscus fragilis, Hibiscus rosa-sinensis, Hibiscus schizopetalus, Hibiscus syriacus), Hordeum* (such as *Hordeum murinum and Hordeum vulgare), Humulus scandens*, *Hydrangea paniculata, Hypericum perforatum*, *Hypochaeris brasiliensis, Ilex* (such as *Ilex aquifolium, Ilex vomitoria), Ipomoea* (such as *Ipomoea fistulosa, Ipomoea purpurea), Iva annua, Jacaranda mimosifolia, Juglans (Juglans regia), Juniperus ashei, Koelreuteria bipinnata, Lactuca serriola, Lagerstroemia* (such as *Lagerstroemia indica), Lathyrus* (such as *Lathyrus cicera, Lathyrus clymenum, Lathyrus sativus), Laurus* (such as *Laurus nobilis), Lavandula* (such as *Lavandula* × *heterophylla, Lavandula* × *intermedia, Lavandula angustifolia, Lavandula dentata, Lavandula latifolia, Lavandula stoechas, Lavatera cretica), Leonurus sibiricus, Lepidium* (such as *Lepidium auriculatum, Lepidium didymium), Leptospermum laevigatum, Ligustrum* (such as *Ligustrum lucidum, Ligustrum sinense, Ligustrum virginicum), Liquidambar styraciflua, Liriodendron tulipifera, Lobularia maritima, Lolium* (such as *Lolium multiflorum, Lolium perenne, Lolium temulentum), Lonicera* (such as *Lonicera implexa, Lonicera japonica), Ludwigia grandiflora, Lupinus* (such as *Lupinus aridorum, Lupinus villosus), Magnolia* (such as *Magnolia grandiflora, Magnolia* × *soulangeana), Mallotus paniculatus, Malva parviflora, Marrubium vulgare, Medicago* (such as *Medicago arborea, Medicago polymorpha, Medicago sativa), Melicope ternata, Melicytus ramiflorus, Melilotus* (such as *Melilotus albus, Melilotus albus* var. *Annuus, Melilotus indicus, Melilotus officinalis), Melissa officinalis, Mentha, Merremia macrocalyx, Meryta* sinclairii, *Metrosideros* (such as *Metrosideros excelsa, Metrosideros kermadecensis), Mimosa, Modiola caroliniana, Montiastrum lineare*, *Morus* (such as *Morus alba, Morus nigra, Morus rubra), Myoporum* (such as *Myoporum insulare, Myoporum laetum), Myrtus communis, Nandina domestica, Neptunia lutea, Nerium oleander, Nicotiana* (such as *Nicotiana benthamiana, Nicotiana clevelandii*, *Nicotiana glauca, Nicotiana tabacum),* Ocimum *basilicum, Oenanthe sarmentosa, Oenothera elata, Olea* (such as *Olea europaea, Olea europaea* subsp. *Sylvestris), Origanum majorana, Osteospermum ecklonis, Osteospermum fruticosum, Panicum acuminatum, Parthenocissus tricuspidata, Paspalum* (such as *Paspalum dilatatum, Paspalum regnellii, Paspalum urvillei), Passiflora foetida, Pelargonium* (such as *Pelargonium fragrans, Pelargonium graveolens, Pelargonium* × *hortorum), Pennisetum* (such as *Pennisetum clandestinum*, *Pennisetum glaucum),* Periwinkle (common name), *Perovskia abrotanoides, Persea americana, Persicaria* (such as *Persicaria lapathifolia, Persicaria maculosa), Phagnalon* (such as *Phagnalon saxatile), Phalaris* (such as *Phalaris angusta, Phalaris minor, Phalaris paradoxa), Phillyrea* (such as *Phillyrea angustifolia, Phillyrea latifolia), Phleum pratense, Phlomis* (such as *Phlomisfruticosa, Phlomis italica), Phoenix* (such as *Phoenix reclinata, Phoenix roebelenii), Phormium* (such as *Phormium colensoi, Phormium tenax*), *Photinia arbutifolia, Pinus taeda, Pistacia vera, Pittosporum* (such as *Pittosporum crassifolium, Pittosporum eugenioides, Pittosporum tenuifolium, Pittosporum umbellatum), Plantago lanceolata, Platanus* (such as *Platanus occidentalis), Pluchea odorata, Poa annua, Polygala* (such as *Polygala myrtifolia, Polygala* × *dalmaisiana, Polygala* × *grandiflora nana), Polygonum arenastrum, Poncirus trifoliata, Populusfremontii, Portulaca oleracea, Prunus* (such as *Prunus americana, Prunus angustifolia, Prunus armeniaca, Prunus avium, Prunus cerasifera, Prunus cerasifera* × *P. salicina, Prunus cerasifera* × *P. munsoniana, Prunus cerasus, Prunus domestica, Prunus dulcis, Prunus dulcis* × *P. webbii, Prunus hortulana, Prunus laurocerasus, Prunus mexicana, Prunus mume, Prunus munsoniana, Prunus persica, Prunus persica* × *P. webbii, Prunus salicina, Prunus salicina* × *(P. salicina* × *P. cerasifera*), *Prunus serotina, Prunus serrulata, Prunus* simonii, *Prunus* simonii × *P. salicina* × *P. cerasifera* × *P. munsoniana* , *Prunus webbii, (Prunus salicina* × *P. angustifolia)* × (*P. salicina* × *P. munsoniana)), Psidium, Pteridium aquilinum, Pyrus* (such as *Pyrus pyrifolia), Quercus* (such as *Quercus agrifolia, Quercus alba, Quercus coccinea, Quercus falcata, Quercus ilex, Quercus imbricaria, Quercus incana, Quercus laevis, Quercus laurifolia, Quercus lobata, Quercus macrocarpa, Quercus nigra, Quercus palustris, Quercus phellos, Quercus pubescens, Quercus robur, Quercus rubra, Quercus shumardii, Quercus suber, Quercus velutina, Quercus virginiana), Ranunculus repens repens, Ratibida columnifera, Reseda odorata, Retama monosperma, Rhamnus (Rhamnus alaternus), Rheum rhaponticum, Rhus (Rhus diversiloba), Richardia, Robinia pseudoacacia, Rosa* (such as *Rosa californica, Rosa canina), Rubus* (such as *Rubus fruticosus, Rubus hedycarpus* subsp. *Procerus, Rubus rigidus, Rubus ulmifolius, Rubus ursinus, Rubus vitifolius), Rumex crispus, Ruta chalepensis, Salix* (such as *Salix laevigata, Salix lasiolepis), Salsola kali* subsp. *Tragus, Salvia* (such as *Salvia apiana, Salvia mellifera, Salvia officinalis, Salvia Rosmarinus), Sambucus* (such as *Sambucus canadensis, Sambucus cerulea, Sambucus nigra), Santolina* (such as *Santolina chamaecyparissus, Santolina magonica), Sapindus saponaria, Sassafras* (such as *Sassafras albidum), Senecio* (such as *Senecio grisebachii, Senecio vulgaris), Senna secundiflora, Setaria magna, Sida rhombifolia, Silybum marianum, Simmondsia chinensis, Sisymbrium irio, Solanum* (such as *Solanum americanum, Solanum lycopersicum, Solanum melongena), Solidago* (such as *Solidago canadensis, Solidago fistulosa, Solidago virgaurea), Sonchus* (such as *Sonchus asper, Sonchus oleraceus), Sophora secundiflora, Sorghum* (such as *Sorghum halepense, Sorghum* × *drummondii), Spartium (Spartium junceum), Stachys arvensis, Stellaria media, Stewartia pseudocamellia, Strelitzia reginae, Streptocarpus* hybrids, *Swainsona galegifolia, Symphoricarpos albus, Symphyotrichum divaricatum, Syringa vulgaris, Syzygium paniculatum, Talinum paniculatum, Taraxacum officinale, Teline monspessulana, Teucrium capitatum, Trifolium* (such as *Trifolium fragiferum, Trifolium hybridum, Trifolium incarnatum, Trifolium pratense, Trifolium repens, Trifolium repens var.latum), Ulex* (such as *Ulex europaeus, Ulex minor, Ulex parviflorus), Ulmus* (such as *Ulmus americana, Ulmus crassifolia, Ulmus glabra, Ulmus pumila, Ulmus* × *hollandica*), *Umbellularia californica, Urtica* (such as *Urtica dioica, Urtica dioica* subsp. *Gracilis, Urtica urens), Vaccinium* (such as *Vaccinium ashei, Vaccinium corymbosum, Vaccinium corymbosum* × *V. angustifolium* hybrid, *Vaccinium darrowii, Vaccinium virgatum), Verbena litoralis, Vernonia, Veronica* (such as *Veronica persica), Viburnum tinus, Vica* (such as *Vicia faba, Vicia ludoviciana, Vicia monantha, Vicia sativa), Vinca (Vinca major, Vinca minor), Vitis (Vitex agnus-castus, Vitex lucens, Vitis acerifolia, Vitis aestivalis, Vitis aestivalis* hybrid, *Vitis aestivalis* var. *Smalliana, Vitis aestivalis* var. *smalliana* × V. *simpsonii, Vitis arizonica, Vitis arizonica* hybrid, *Vitis arizonica* × *V. vinifera, Vitis arizonica*/*candicans, Vitis arizonica*/*candicans* × *V. rupestris, Vitis arizonica*/*girdiana, Vitis arizonica*/*girdiana* × *V. rupestris, Vitis berlandieri, Vitis bloodwothiana, Vitis bourquiniana, Vitis californica, Vitis candicans, Vitis champinii* × (*V. solonis* × *V. othello), Vitis cinerea, Vitis cinerea* × *V. berlandieri, Vitis* × *doaniana, Vitis girdiana, Vitis* hybrids, *Vitis labrusca, Vitis labrusca* × *V. vinifera, Vitis lincecumii, Vitis monticola, Vitis munsoniana, Vitis muscadina, Vitis nesbittiana, Vitis palmata, Vitis riparia, Vitis rotundifolia, Vitis rotundifolia* × *V. rupestris, Vitis rufotomentosa, Vitis rupestris, Vitis shuttleworthii, Vitis simpsonii, Vitis tiliaefolia, Vitis treleasei, Vitis vinifera, Vitis vinifera* hybrid, *Vitis vulpina, Vitis* × *champinii, Vitis* × *cinerea* var. *Helleri* × *V. vulpine), Vulpia myuros, Westringia* (such as *Westringia fruticosa, Westringia glabra), Wisteria frutescens, Xanthium* (such as *Xanthium orientale, Xanthium spinosum, and Xanthium strumarium).*

In preferred embodiments of the method for treating a disease and/or increasing resistance against an infection in a plant, the administering comprises applying the peptide, complex and/or plant protection agent directly to the plant and/or applying the peptide, complex and/or the plant protection agent to the environment surrounding the plant.

In the context of the invention, "applying" the peptide, complex and/or plant protection agent refers to contacting the peptide, complex and/or plant protection agent with a plant, plant part (referred to as "directly" applying), and/or the plant's surrounding environment (e.g. the soil) such that the peptide, complex and/or plant protection agent can have an effect on that plant and/or plant part. Preferably, the application includes contacting the peptide, complex and/or plant protection agent directly with a plant, plant part, and/or the plant's surrounding environment (e.g. the soil).

In preferred embodiments of the invention, the peptide, complex and/or plant protection agent, preferably the peptide, is administered into the plant, preferably into the xylem vessel. This ensures that the plant protection agent is in the plant, preferably in the xylem vessel. In preferred embodiments, the plant protection agent, preferably the peptide is in the plant, preferably in the xylem vessel.

In preferred embodiments, applying the peptide, complex and/or plant protection agent directly to the plant comprises injecting the peptide, complex and/or plant protection agent into the plant. In preferred embodiments the injection is an injection into the plants' trunk, roots, leaves, vascular system, and/or stem.

In further preferred embodiments of the invention, said injection is a trunk injection and/or stem injection, preferably a trunk injection. In preferred embodiments, the plant is an olive, citrus, almond and/or grapevine. In preferred embodiments of the invention applying the peptide, complex and/or plant protection agent directly to the plant comprises a trunk injection, wherein the plant is an olive, citrus, almond and/or grapevine. In preferred embodiments of the invention, applying the peptide, complex and/or plant protection agent directly to the plant comprises applying the peptide, complex and/or plant protection agent to the surface of the plant such as its roots, leaves, and/or stem.

In preferred embodiments, administering the peptide, complex and/or plant protection agent to the environment surrounding the plant comprises applying the peptide, complex and/or plant protection agent to the soil, air, or water surrounding the plant.

Preferably, the peptide, complex and/or plant protection agent is sprayed in the form of a liquid or a dry powder, dust, granules, microgranules, pellets, wettable powder, flowable powder, emulsion, microcapsules, oil, gel, paste or aerosol.

In preferred embodiments of the invention, the effect of the peptide, complex and/or plant protection agent is enhanced by mixing the peptide, complex and/or plant protection agent with suitable adjuvants. In preferred embodiments, the peptide, complex and/or plant protection agent is formulated as a dry powder, which can be mixed with water and other components to form a liquid product.

In preferred embodiments, the peptide, complex and/or plant protection agent is contacted with a plant part. In further preferred embodiments, the peptide, complex and/or plant protection agent is contacted with one or more roots of the plant. Preferably, the peptide, complex and/or plant protection agent is be applied directly to the roots, e.g., by spraying or pouring onto the roots. Preferably, the peptide, complex and/or plant protection agent is applied indirectly to the soil in which the plant roots are growing (i.e., the rhizosphere). Preferably, the peptide, complex and/or plant protection agent is applied to the seeds of the plant prior to or at the time of planting, or to any other part of the plant and/or its surrounding environment.

In preferred embodiments, the method comprises administering the peptide, complex and/or plant protection agent into a tank connected to an irrigation system used for supplying water, fertilizers, or other liquid compositions to a crop, orchard or field.

Thus, the plant and/or soil surrounding the plant can be treated with the peptide, complex and/or plant protection agent via, for example, soil injection, soil drenching, or using a center pivot irrigation system, or with a spray over the seed furrow, or with sprinklers or drip irrigators. Advantageously, the method is suitable for treating hundreds of acres of a plantation, crop, orchard or field at one time.

In preferred embodiments, the method for treating a disease and/or increasing resistance against an infection in a plant, comprises manufacturing of the peptide. Preferably, the manufacturing of the peptide comprises conducting a method of any other aspect of the invention.

**In an eleventh aspect,** the invention pertains to a use of a plant protection agent according to any aspect of the invention in the treatment of a disease and/or increasing resistance against infection in a plant, preferably, wherein the disease and/or infection is a fungal and/or bacterial disease and/or infection.

In preferred embodiments, the plant is a plant according to any other aspect of the invention. Preferably, the disease and /or infection is an infection according to any other aspect of the invention. In preferred embodiments of the invention, the bacterial infection is an infection with a *Xylella* bacterium, preferably, wherein the *Xylella* bacterium is *Xylella fastidiosa.*

In preferred embodiments, the plant protection agent can be used to supplement other treatments in a plant.

**In a twelfth aspect,** the invention pertains to a plant protection composition comprising at least one peptide or a salt thereof according to any one of the other aspects of the invention and a phytopharmaceutically acceptable carrier and/or excipient.

The plant protection composition preferably replaces the plant protection agent or the peptide of the invention in any of the other aspects or embodiments of the invention. Preferably, the plant protection composition replaces the peptide, complex and/or plant protection agent in any of the aspects or embodiments of the invention that pertain to i) a use of said peptide, complex and/or plant protection or ii) a method for treating a disease and/or increasing resistance against an infection in a plant, wherein the method comprising administering to the plant the peptide, complex and/or plant protection.

The term "phytopharmaceutically acceptable" as used herein inter alia in connection with compound forms such as salts, hydrates, solvates, as well as in connection with carriers (such as vehicles, supports, solvents, etc.), is consistent with the art and means not deleterious to the recipient plant, such as not producing any adverse effects when applied to a plant or to an organ of the plant, and where applicable, compatible with any other ingredients of a phytopharmaceutical composition. Phytopharmaceutically acceptable acid and base addition salts are meant to comprise the phytopharmaceutically active non-toxic acid and base addition salt forms which the compound is able to form. Such salts can conveniently be obtained by treating the base form of a compound with an appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, ascorbic, gallic, hydroxyacetic, lactic, pyruvic, malonic, succinic (i.e. butanedioic acid), maleic, fumaric, dehydroascorbic, malic, tartaric, citric, methanesulfonic, ethanesulfonic, benzenesulfonic, p-toluenesulfonic, cyclamic, salicylic, p- aminosalicylic, pamoic, coumaric, ferulic, caffeic, sinapic, cinnamic, resveratrol, catechin, 3- hydroxyl-antranilic, L-DOPA, melanin, eumelanin, lignols, vanillic, vanillin and the like acids. Conversely said salt forms can be converted by treatment with an appropriate base into the free base form. A compound containing an acidic proton (such as cellobionic acid, aldonic acids) may also be converted into its non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, aluminum salts, zinc salts, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-w-butylamine, pyrrolidine, piperidine, morpholine, trimethylamine, triethylamine, tripropylamine, quinuclidine, pyridine, quinoline and isoquinoline; the benzathine, A-methyl-D-glucamine, hydrabamine salts, and salts with amino acids such as, for example, arginine, lysine and the like. Conversely the salt form can be converted by treatment with acid into the free acid form. The term solvate comprises the hydrates and solvent addition forms which the compound is able to form, as well as the salts thereof. Examples of such forms are, e.g., hydrates, alcoholates and the like.

**In a thirteenth aspect,** the invention pertains to the use of a peptide according to any other aspect of the invention as a plant protection agent against *Xylella.* Preferably, the plant protection agent is the plant protection agent according to any of the other aspects of the invention. Preferably, *Xylella* refers to a *Xylel*la infection as defined in any other aspects of the invention.

Additionally, the invention pertains to the following itemized embodiments:
Item 1: A method for treating a disease and/or increasing resistance against an *Xylella* infection in a plant, wherein said method comprises administering to the plant a plant protection agent that comprises:
   a peptide that comprises a chain of amino acids with the sequence X₁X₂X₃X₄X₅X₆X₇X₈X₉X₁₀X₁₁X₁₂X₁₃X₁₄X₁₅,
   wherein X is an amino acid or no amino acid (absent) according to the following definitions:
      - X₁ is absent or L-Dap;
      - X₂ is *N*-methyl-L-Val;
      - X₃ is D-*allo*-Thr;
      - X₄ is D-Ala;
      - X₅ is β-OH-L-*allo*-Asp;
      - X₆ is β-OH-L-Phe;
      - X₇ is D-*allo*-Ile;
      - X₈ is L-Ser;
      - X₉ is D-Lys;
      - X₁₀ is L-Dap;
      - X₁₀ is D-Val, D-*allo*-Ile or D-Leu;
      - X₁₂ is β-OH-L-*allo*-Asp;
      - X₁₃ is L-Leu;
      - X₁₄ is β-OH-L-*allo*-Asp;
      - X₁₅ is β-OH-L-Ile;
         wherein L-Dap is L-2,3-Diaminopropionic acid;
         wherein sequential amino acids are connected by a carboxamide bond, and wherein a fatty acid group R is bound covalently to an amino acid of the chain of amino acids of the peptide; and
         wherein the amino acids X₃X₄X₅X₆X₇X₈X₉X₁₀X₁₁X₁₂X₁₃X₁₄X₁₅ of the peptide form a cycle and the non-side chain carboxyl group of the amino acid X₁₅ is covalently connected to the amino acid X₃,
   or wherein the plant protection agent comprises:
      a complex consisting of said peptide and a metal atom;
      a nucleic acid sequence encoding for an NRPS able to synthesize the peptide or parts of the peptide; and/or
      a cell, wherein the cell comprises the nucleic acid sequence encoding for the NRPS, and/or wherein the cell comprises a vector comprising the nucleic acid sequence encoding for the NRPS.
Item 2: The method of item 1, wherein the chain of amino acids is:
   X₁~X₂~X₃~X₄~X₅~X₆~X₇~X₈~X₉^{∗}X₁₀~X₁₁~X₁₂~X₁₃~X₁₄~X₁₅ or
   X₁~X₂~X₃~X₄~X₅~X₆~X₇~X₈~X₉~X₁₀~X₁₁~X₁₂~X₁₃~X₁₄~X₁₅,

   wherein ~ between two adjacent amino acids indicates that the two adjacent amino acids are covalently connected by a peptide bond connecting the non-side chain carboxyl group of the first of the two adjacent amino acids with the non-side chain amino group of the second of the two adjacent amino acids.
   wherein * between two adjacent amino acids indicates that the two adjacent amino acids are covalently connected by an isopeptide bond connecting the non-side chain carboxyl group of the first of the two adjacent amino acid with a side chain amino group of the second of the two adjacent amino acids.
Item 3: The method of any one of items 1 or 2, wherein the fatty acid group R is selected from a compound with the structure (I) 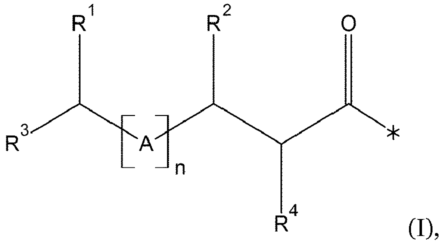
   wherein each R¹, R³, and R⁴ is independently selected from the group consisting of: H, G₁₋₆ alkyl, C₂₋₆ alkenyl, C₂₋₆ alkynyl and 3 to 6 membered cycloalkyl, wherein each H may independently be substituted by Cl and/or F, preferably wherein each R¹ is independently selected from the group -H, -CH₃, *tert*-butyl, isobutyl and cyclopropyl,
   wherein R² is selected from the group consisting of -H, -CN, azido, -NO₂, -NH₂, -OH, -OCH₃₋ₓFₓ, -SH, -SCH₃₋ₓFₓ, -CH₃₋ₓFₓ (wherein x is 0 to 3), 3 to 6 membered cycloalkyl, 3 to 6 membered heterocyclyl, 3 to 6 membered aryl, and 3 to 6 membered heteroaryl, wherein each hydrogen atom in the 3 to 6 membered cycloalkyl, 3 to 6 membered heterocyclyl, 3 to 6 membered aryl or 3 to 6 membered heteroaryl may independently be substituted by a substituent selected from the group of -CN, halogen (preferably wherein the halogen is F and/or Cl), azido, -NO₂, -NH₂, -OH, - OCH₃₋ₓFₓ, -SH, -SCH₃₋ₓFₓ, and -CH₃₋ₓFₓ, wherein x is 0 to 3, preferably wherein the heteroaryl or heterocycyl contains at least one heteroatom selected from the group of nitrogen, oxygen, and/or sulphur,
   wherein each of the A is independently selected from the group of -(CH₂)-, -(CH=CH)-, -(C=C)-, -(C=O)-, wherein each hydrogen atom of -(CH₂)- and -(CH=CH)- may independently be substituted with halogen, (preferably wherein the halogen is Cl, and/or F), and/or CH₃₋ₓFₓ, wherein x is 0 to 3;
   wherein n is 0 to 20,
Item 4: The method of item 3, wherein
   n is 5;
   R¹, R³, and R⁴ are each independently selected from -CH₃ or -H;
   R² is -NH₂ or -OH; and
   A is -(CH₂)-.
Item 5: The method of any one of items 1 to 4, wherein the fatty acid group R is selected from a compound with the structure 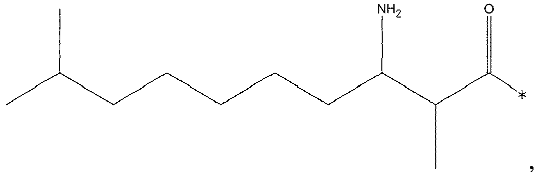 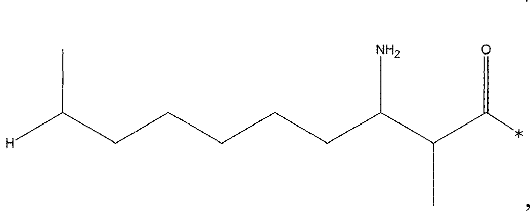 or 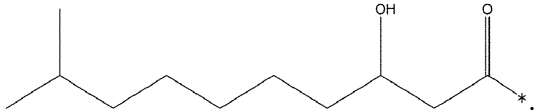 wherein the fatty acid group R is bound to the chain of amino acids via a peptide bond.
Item 6: The method of any one of items 1 to 4, wherein the fatty acid group R is bound to the amino acid of the chain of amino acids at the X position with the lowest integer.
Item 7: The method of anyone one of items 1 to 6, wherein the fatty acid group R is bound to the chain of amino acids via a carboxamide bond
Item 8: The method of any one of items 1 to 7, wherein the peptide is any one of the molecules with the structure (II-VII) 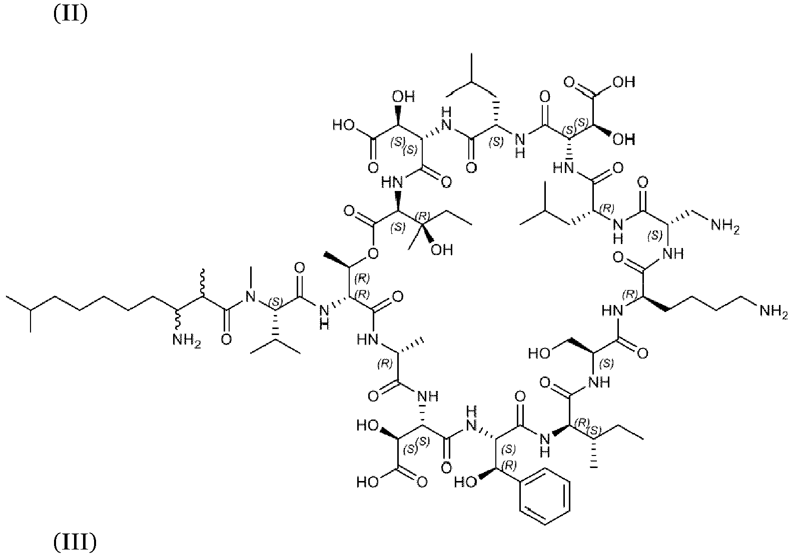 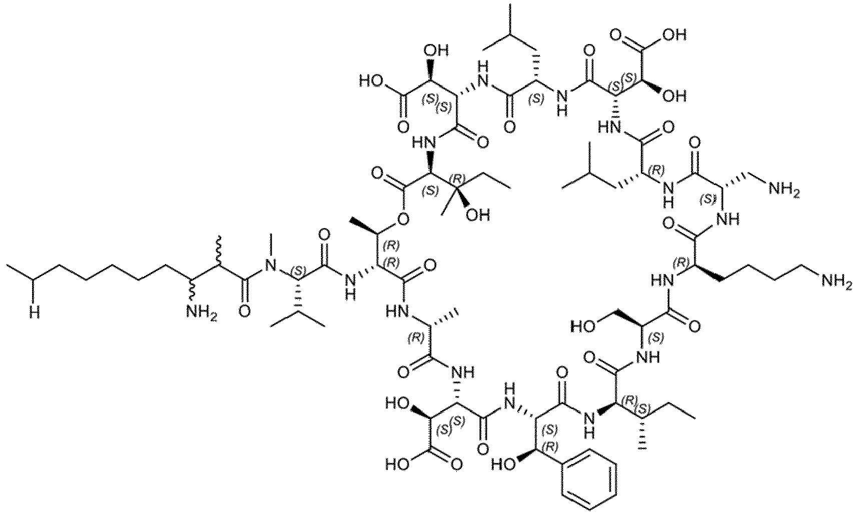 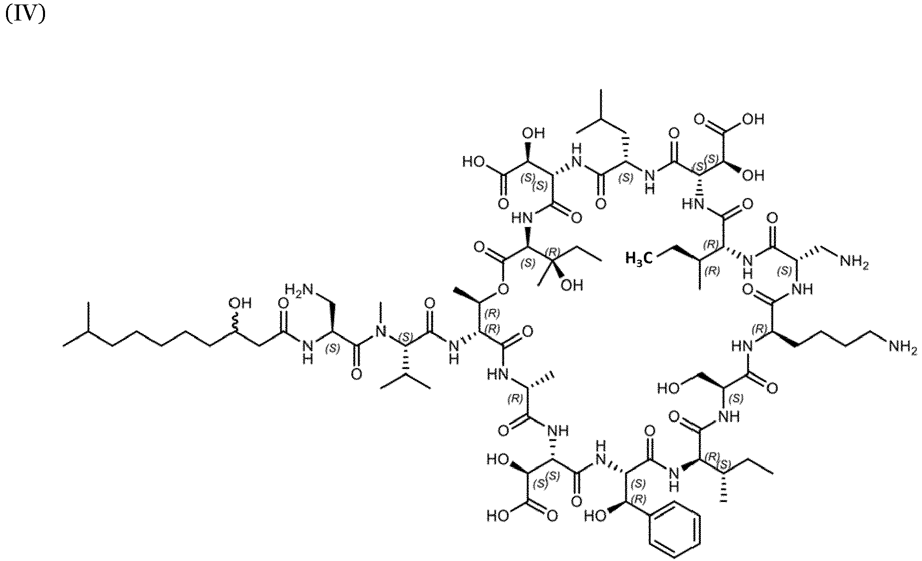 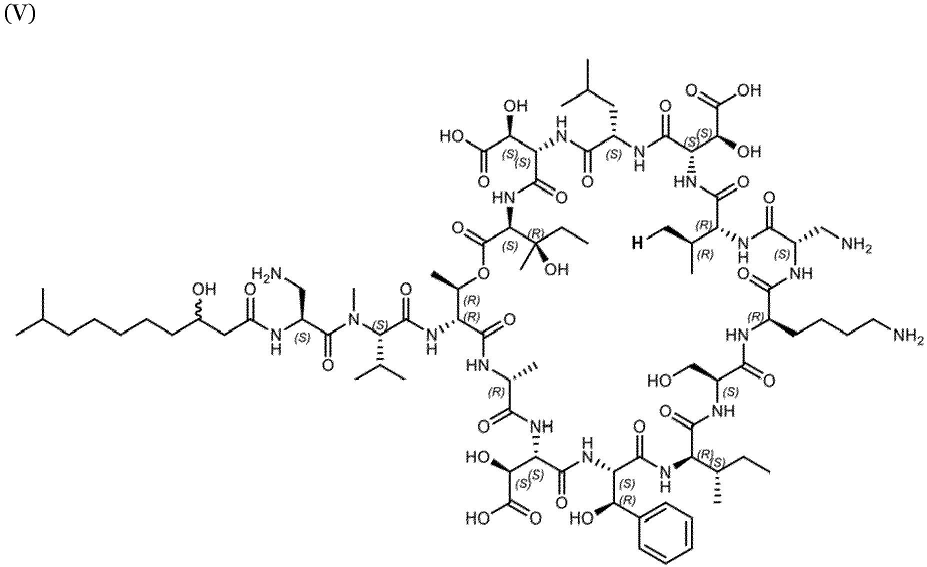 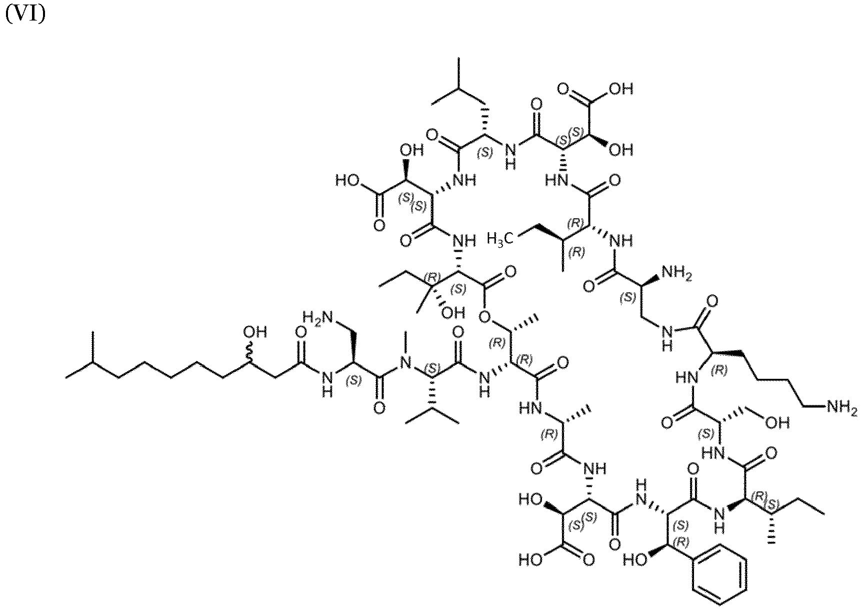 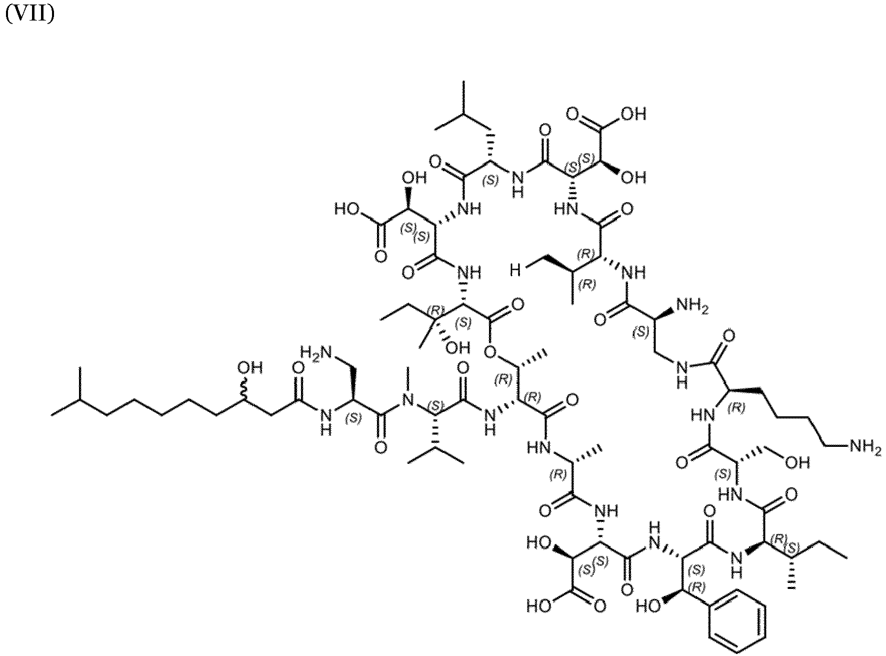
Item 9: The method of any one of items 1 to 8, wherein the complex consists of the peptide and a metal atom selected from the group of Fe, Cu, and Zn, preferably Fe.
Item 10: The method of any one of items 1 to 9, wherein the *Xylella* infection is an infection with a *Xylella* bacterium, preferably, wherein the *Xylella* bacterium is *Xylella fastidiosa.*
Item 11: The method of any one of items 1 to 10, wherein administering comprises applying the plant protection agent directly to the plant and/or applying the plant protection agent to the environment surrounding the plant.
Item 12: The method of any one of items 1 to 11, wherein the plant protection agent is administered into the plant, preferably into the xylem vessel.
Item 13: Use of a plant protection agent, in the treatment of a disease and/or increasing resistance against a *Xylella* infection in a plant, wherein the plant protection agent comprises
   a peptide that comprises a chain of amino acids with the sequence X₁X₂X₃X₄X₅X₆X₇X₈X₉X₁₀X₁₁X₁₂X₁₃X₁₄X₁₅,
      wherein X is an amino acid or no amino acid (absent) according to the following definitions:
         - X₁ is absent or L-Dap;
         - X₂ is *N*-methyl-L-Val;
         - X₃ is D-*allo*-Thr;
         - X₄ is D-Ala;
         - X₅ is β-OH-L-*allo*-Asp;
         - X₆ is β-OH-L-Phe;
         - X₇ is D-*allo*-Ile;
         - X₈ is L-Ser;
         - X₉ is D-Lys;
         - X₁₀ is L-Dap;
         - X₁₁ is D-Val, D-*allo*-Ile or D-Leu;
         - X₁₂ is β-OH-L-*allo*-Asp;
         - X₁₃ is L-Leu;
         - X₁₄ is β-OH-L-*allo*-Asp;
         - X₁₅ is β-OH-L-Ile;
      wherein L-Dap is L-2,3-Diaminopropionic acid;
      wherein sequential amino acids are connected by a carboxamide bond, and wherein a fatty acid group R is bound covalently to an amino acid of the chain of amino acids of the peptide;
      wherein the amino acids X₃X₄X₅X₆X₇X₈X₉X₁₀X₁₁X₁₂X₁₃X₁₄X₁₅ of the peptide form a cycle and the non-side chain carboxyl group of the amino acid X₁₅ is covalently connected to the amino acid X₃;
   or wherein the plant protection agent comprises;
   a complex consisting of said peptide and a metal atom;
   a nucleic acid sequence encoding for an NRPS able to synthesize the peptide or parts of the peptide and/or
   a cell, wherein the cell comprises the nucleic acid sequence encoding for the NRPS, and/or wherein the cell comprises a vector comprising the nucleic acid sequence encoding for the NRPS.
Item 14: A method of manufacturing a peptide that comprises a chain of amino acids with the sequence X₁X₂X₃X₄X₅X₆X₇X₈X₉X₁₀X₁₁X₁₂X₁₃X₁₄X₁₅,
   wherein X is an amino acid or no amino acid (absent) according to the following definitions:
      - X₁ is absent or L-Dap;
      - X₂ is N-methyl-L-Val;
      - X₃ is D-*allo*-Thr;
      - X₄ is D-Ala;
      - X₅ is β-OH-L-*allo*-Asp;
      - X₆ is β-OH-L-Phe;
      - X₇ is D-*allo*-Ile;
      - X₈ is L-Ser;
      - X₉ is D-Lys;
      - X₁₀ is L-Dap;
      - X₁₀ is D-Val, D-*allo*-Ile or D-Leu;
      - X₁₂ is β-OH-L-*allo*-Asp;
      - X₁₃ is L-Leu;
      - X₁₄ is β-OH-L-*allo*-Asp;
      - X₁₅ is β-OH-L-Ile;
         wherein L-Dap is L-2,3-Diaminopropionic acid;
         wherein sequential amino acids are connected by a carboxamide bond, and wherein a fatty acid group R is bound covalently to an amino acid of the chain of amino acids of the peptide;
         wherein the amino acids X₃X₄X₅X₆X₇X₈X₉X₁₀X₁₁X₁₂X₁₃X₁₄X₁₅ of the peptide form a cycle and the non-side chain carboxyl group of the amino acid X₁₅ is covalently connected to the amino acid X₃;
   wherein the method comprises a recombinant and/or a chemical synthesis.
Item 15: A cell, comprising a recombinant nucleic acid sequence and/or comprising a recombinant vector comprising a nucleic acid sequence encoding for a peptide, wherein the peptide comprises a chain of amino acids with the sequence X₁X₂X₃X₄X₅X₆X₇X₈X₉X₁₀X₁₁X₁₂X₁₃X₁₄X₁₅;
   wherein X is an amino acid or no amino acid (absent) according to the following definitions:
      - X₁ is absent or L-Dap,
      - X₂ is *N*-methyl-L-Val
      - X₃ is D-*allo*-Thr
      - X₄ is D-Ala
      - X₅ is β-OH-L-*allo*-Asp
      - X₆ is β-OH-L-Phe
      - X₇ is D-*allo*-Ile
      - X₈ is L-Ser
      - X₉ is D-Lys
      - X₁₀ is L-Dap
      - X₁₁ is D-Val, D-*allo*-Ile or D-Leu
      - X₁₂ is β-OH-L-*allo*-Asp
      - X₁₃ is L-Leu
      - X₁₄ is β-OH-L-*allo*-Asp
      - X₁₅ is β-OH-L-Ile
   wherein L-Dap is L-2,3-Diaminopropionic acid,
   wherein sequential amino acids are connected by a carboxamide bond, and wherein a fatty acid group R is bound covalently to an amino acid of the chain of amino acids of the peptide
   wherein the amino acids X₃X₄X₅X₆X₇X₈X₉X₁₀X₁₁X₁₂X₁₃X₁₄X₁₅ of the peptide form a cycle and the non-side chain carboxyl group of the amino acid X₁₅ is covalently connected to the amino acid X₃.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND SEQUENCES

The figures show:
**Figure 1****:** shows chemical structures of the Chitinopeptins A-D.

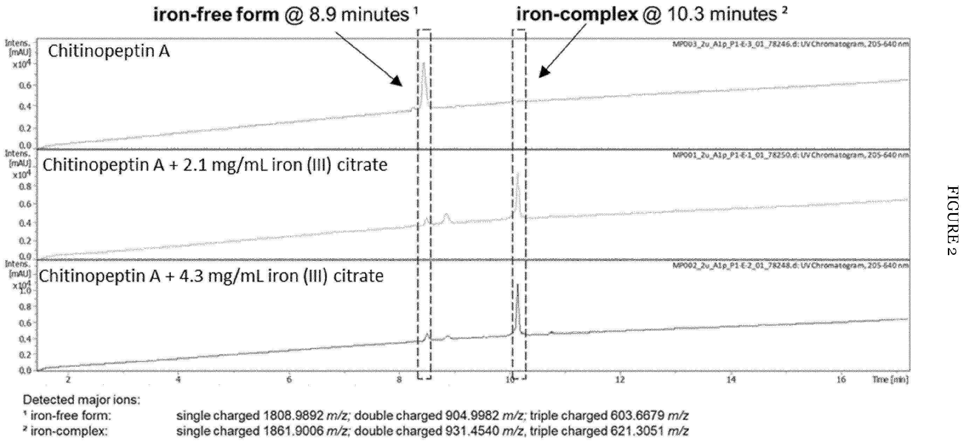

**Figure 2****:** shows LTV-chromatograms of Chitinopeptin A preparations. The same preparations were tested in the *Xylella fastidiosa* bioassay for determining the minimum inhibitory concentration (Table 1). (Top): Chitinopeptin A; UV-spectrum shows a peak at a retention time of 8.9 minutes. Acquisition of corresponding mass spectra allowed the observation of major ions of iron-free Chitinopeptin A (1808.9882 m/z, 904.9982 m/z and 603.6679 m/z). (Middle and Bottom): Chitinopeptin A supplemented with 2.1 mg/mL (middle) or 4.3 mg/mL iron(III)citrate (bottom): UV-spectrums shows a peak at a retention time of 10.3 minutes. The corresponding mass spectra of these peaks show major ions of iron-coordinating Chitinopeptin A (1861.9006 m/z, 931.4540 m/z and 621.3051 m/z).

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Culturing and Extraction of Chitinopeptins

Bacterial strains and culture conditions: All strains were purchased from Deutsche Sammlung von Mikroorganismen und Zellkulturen (DMSZ) and Korean Collection for Type Cultures (KCTC). *C. eiseniae* DSM 22224 and *C. flava* KCTC 62435 were inoculated from plate (R2A, (HiMedia Laboratories, LLC), (32)) in 300-mL Erlenmeyer flasks filled with 100 mL R2A and incubated at 28 °C with agitation at 180 rpm for 3 days. This was followed by 20 L fermentations (separated in 500 mL culture volume per 2 L flasks) in 3018-medium (1 g/L yeast extract, 5 g/L Casitone, pH 7.0, 24 mM *N-acetylglucosamine* added after autoclaving) inoculated with 2% (vol/vol) pre-culture, incubated at 28 °C with agitation at 180 rpm for 4 days. Subsequently, the cultures were freeze-dried. Dried cultures were extracted with onetime culture volume MeOH.

Chitinopeptin Extraction: The extracts were evaporated to dryness using rotary evaporation under reduced pressure, resuspended in 3 L of 10% MeOH/H₂O, and loaded onto a XAD16N column (1 Lbed volume). Step-wise elution with 10%, 40%, 60%, 80%, and 100% MeOH (two-times bed volume each) was performed. The 80% and 100% fractions containing chitinopeptins were subjected to preparative followed by semi-preparative reverse-phase highperformance liquid chromatography (RP-HPLC) on C18 columns (preparative HPLC: Synergi 4 µm Fusion-RP 80 Å [250 × 21.2 mm]; semi-preparative-HPLC: Synergi 4 µm Fusion-RP 80 Å [250 × 10 mm], mobile phase: CH₃CN/H₂O + 0.1% HCOOH) using linear gradients of 5% to 95% organic in 40 min. Final purification was achieved using UHPLC on a ACQUITY UPLC BEH C18 column (130 Å, 1.7 µm, 100 × 2.1 mm) with the same mobile phase and a linear gradient of 30% to 60% organic in 18 min. After each step, fractions containing chitinopeptins were evaporated to dryness using a high performance evaporator (Genevac HT-12).

### Example 2: Coordination behavior of Chitinopeptin A

The inventors further studied the complex formation of the chitinopeptins. In such an experiment, they exposed Chitinopeptin A to an aqueous solution of iron(III) citrate (2.1 mg/ml and 4.3 mg/ml) and characterized the samples by conducting UHPLC-QTOF-HR-MS and MS/MS measurements similarly as described in (2, 7). For the results, compare to Figure 2.

Briefly, acquisition of UHPLC-QTOF-HRMS and MS/MS data was carried out using a quadrupole time-of-flight mass spectrometer (LC-QTOF maXisII Bruker Daltronics, Bremen, Germany) in line with an Agilent 1290 Infinity LC system equipped with a BEH C18 column (130 Å, 100 mm × 2.1 mm, 1.7 µm, Waters, Germany). A linear gradient of water and acetonitrile, both supplemented with 0.1% formic acid, at a constant flow of 600 µL/min was used to separate the analytes by reversed phase chromatography, while UV detection was done in the 205-640 nm range.

Under these conditions, pure Chitinopeptin A could be detected in a reference sample without iron(III) citrate addition, by using by UV detection at a retention time of 8.9 minutes. At the same time, the mass spectra confirmed the presence of Chitinopeptin A with peaks of its mainions at 1808.9892 m/z for single charged, 904.9982 m/z for double charged, and 603.6679 m/z for triple charged species. The mass spectra of the samples containing iron (III) citrate on the other hand also show peaks of the major ions of a Chitinopeptin A species that is coordinating iron (III), namely at 1861.9006 m/z for single charged, 931.4540 m/z for double charged, and 621.3051 m/z for triple charged species. Similarly, the this complex can also be detected in the UV-chromatograms (Figure 2 middle and bottom), in which the iron(III) complex is detected at a retention time of 10.3 minutes.

### Example 3: Antimicrobial effect of Chitinopeptin A

In the present application, the inventors show the effect of Chitinopeptin A on *Xylella fastidiosa.* The minimum inhibitory concentration (MIC) of iron-free and iron-saturated Chitinopeptin A was determined by micro-broth-dilution assays in 96-well plates. In these experiments, the compound was dissolved in dimethyl sulfoxide (DMSO, Carl Roth GmbH + Co., Karlsruhe, Germany) and supplemented with either 0 mg/mL, 2.1 mg/mL or 4.3 mg/mL iron(III)citrate (A10276, Alfa Aesar, Kandel, Germany).

All preparations were tested against *Xylella fastidiosa* in a 12-point dilution series ranging from 32 - 0.016 µg/mL in triplicate. The strain *Xylella fastidiosa* DSM10026 was obtained from the German Collection of Microorganisms and Cell Cultures GmbH. Working cryostocks were stored in 50% glycerol at -80°C. Cyro-preserved cells were streaked on PD2 agar medium (16) and incubated for 7 days at 28°C. Grown colonies were scraped off and re-streaked on a fresh PD2 plates to remove residual glycerol. After incubation (7 days at 28°C) the assay solution was prepared by dissolving *Xylella fastidiosa* colonies in PD3 broth medium (PD2 medium without agar). The OD600 of the assay inoculum of adjusted to 0.05 by diluting with PD3 broth medium + 1g/L Tween 80. As positive control, a dilution series of gentamicin (Merck KGaA, Darmstadt, Germany) and cecropin A (GenScript, Piscataway, NJ, USA) was prepared (64 - 0.03 µg/mL and 50 - 0.02 µM). Cell suspensions without test sample or antibiotic control were used as negative controls. Blank tests of DMSO and iron(III)citrate were done. After assay incubation (7 days, 28°C, 180 rpm), the cell viability of *Xylella fastidiosa* was determined by ATP quantification (BacTiter-Glo^{™}, Promega) according to the manufacturer's instructions using a LUMIstar^{®} Omega Microplate Reader (BMG LABTECH GmbH, Ortenberg, Germany). The MIC was defined as lowest concentration of test compound which caused a reduced cell viability of >80% relative to the untreated control. Results are summarized in Table 1.

**Table 1: Minimum Inhibitory Concentration (MIC) of Chitinopeptin A in comparison to the reference compounds Cecropin A and Gentamicin against Xylella fastidiosa DSM10026/ATCC35879.**

| **Preparation** | **MIC (µg mL⁻¹)** | **MIC (µM)** |
|---|---|---|
| Chitinopeptin A | 16-8 | 8.8-4.4 |
| Chitinopeptin A + 2.1 mg/mL iron (III) citrate | 16-8 | 8.8-4.4 |
| Chitinopeptin A + 4.3 mg/mL iron (III) citrate | 16-8 | 8.8-4.4 |
| Cecropin A BR001 | 25 | 6.25 |
| Gentamicin | 4-2 | 8.4-4.2 |

Overall, Chitinopeptin A, regardless of being in a complex with iron(III) or not, did show a minimum inhibitory concentration that is comparable to these commercially available agents. It features a stronger mass-based MIC than Cecropin A. Its molar MIC is comparable to that of Gentamicin.

While the past study of the inventors indicated that the antimicrobial properties of Chitinopeptin A did moderately decrease upon complexation with iron(III), the MIC of Chitinopeptin A against *Xylella fastidiosa* was not affected by the complexation.

### REFERENCES

The references are:
(1) Health, E.Panel o.P.; Bragard, C.; Dehnen-Schmutz, K.; Di Serio, F.; Gonthier, P.; Jacques, M.-A.; Jaques Miret, J.A.; Justesen, A.F.; MacLeod, A.; Magnusson, C.S.; et al. Update of the Scientific Opinion on the risks to plant health posed by Xylella fastidiosa in the EU territory. EFSA Journal 2019, 17, e05665, doi: https://doi.org/10.2903/j.efsa.2019-5665.
(2) Starner, K.; Goh, K.S. Detections of the Neonicotinoid Insecticide Imidacloprid in Surface Waters of Three Agricultural Regions of California, USA, 2010-2011. Bulletin of Environmental Contamination and Toxicology 2012, 88, 316-321, doi:10.1007/s128-011-0515-5.
(3) Lu, C.; Warchol, K.M.; Callahan, R. Sub-lethal exposure to neonicotinoids impaired honey bees winterization before proceeding to colony collapse disorder. Bulletin of Insectology 2014, 67, 125-130.
(4) US20050053584A1
(5) US020180310569A1
(6) Das, M.; Bhowmick, T.S.; Ahern, S.J.; Young, R.; Gonzalez, C.F. Control of Pierce's Disease by Phage. PLOS ONE 2015, 10, e0128902, doi:10.1371/journal.pone.0128902.
(7) Zicca, S.; De Bellis, P.; Masiello, M.; Saponari, M.; Saldarelli, P.; Boscia, D.; Sisto, A. Antagonistic activity of olive endophytic bacteria and of Bacillus spp. strains against Xylella fastidiosa. Microbiological Research 2020, 236, 126467, doi:https://doi.org/10.1016/j.micres.2020.126467.
(8) US20120282233A1
(9) US020090202444A1
(10) WO002021094818A1
(11)Health, E.Panel o.P. Treatment solutions to cure Xylella fastidiosa diseased plants. EFSA Journal 2016, 14, e04456, doi:https://doi.org/10.2903/j.efsa.2016.4456.
(12) Marco, S.; Jianchi, C.; Monica, D.C.; Giuseppe, D.; Nicoletta, P.; Vanessa, M.; Alessia L', A.; Milena, P.; Luigi, Z.; Francesco, M.; et al. A zinc, copper and citric acid biocomplex shows promise for control of Xylella fastidiosa subsp. pauca in olive trees in Apulia region (southern Italy). Phytopathologia Mediterranea 2018, 57, doi:10.14601/Phytopathol_Mediterr-21985.
(13) Baró, A.; Mora, I.; Montesinos, L.; Montesinos, E. Differential Susceptibility of Xylella fastidiosa Strains to Synthetic Bactericidal Peptides. Phytopathology® 2020, 110, 1018-1026, doi:10.1094/PHYTO-12-19-0477-R.
(14) Li, Z.; Gray, D. Effect of five antimicrobial peptides on the growth of Agrobacterium tumefaciens, Escherichia coli and Xylella fastidiosa. VITIS-Journal of Grapevine Research 2015, 42, 95.
(15) Kuzina, L.V.; Miller, T.A.; Cooksey, D.A. In vitro activities of antibiotics and antimicrobial peptides against the plant pathogenic bacterium Xylella fastidiosa. Lett Appl Microbiol 2006, 42, 514-520, doi:10.1111/j.1472-765X.2006.01898.x.
(16) Andersen, P.; Ishida, M.; Momol, E.; Brodbeck, B.; Leite, B.; Momol, M. Influence of Vitis xylem fluid and xylem fluid plus cecropin on growth of Xylella fastidiosa. Vitis 2004, 43.
(17) Moll, L.; Badosa, E.; Planas, M.; Feliu, L.; Montesinos, E.; Bonaterra, A. Antimicrobial Peptides With Antibiofilm Activity Against Xylella fastidiosa. Frontiers in Microbiology 2021, 12, doi:10.3389/fmicb.2021.753874.
(18) Dijksteel, G.S.; Ulrich, M.M.W.; Middelkoop, E.; Boekema, B.K.H.L. Review: Lessons Learned From Clinical Trials Using Antimicrobial Peptides (AMPs). Frontiers in Microbiology 2021, 12, doi:10.3389/fmicb.2021.616979.
(19) Huan, Y.; Kong, Q.; Mou, H.; Yi, H. Antimicrobial Peptides: Classification, Design, Application and Research Progress in Multiple Fields. Frontiers in Microbiology 2020, 11, doi:10.3389/fmicb.2020.582779.
(20) US7151203B2
(21) EP000003885355A1
(22) WO002006069160A3
(23) Bleve, G.; Gallo, A.; Altomare, C.; Vurro, M.; Maiorano, G.; Cardinali, A.; D'Antuono, I.; Marchi, G.; Mita, G. In vitro activity of antimicrobial compounds against Xylella fastidiosa, the causal agent of the olive quick decline syndrome in Apulia (Italy). FEMS Microbiology Letters 2018, 365, doi:10.1093/femsle/fnx281.
(24) WO2018144478A1
(25) US20120282233A1
(26) Evidente, A.; Andolfi, A.; Cimmino, A.; Vurro, M.; Fracchiolla, M.; Charudattan, R. Herbicidal potential of ophiobolins produced by Drechslera gigantea. Journal of agricultural and food chemistry 2006, 54 5, 1779-1783.
(27) Li, S.; Shao, M.-W.; Lu, Y.-H.; Kong, L.-C.; Jiang, D.-H.; Zhang, Y.-L. Phytotoxic and Antibacterial Metabolites from Fusarium proliferatum ZS07 Isolated from the Gut of Long-horned Grasshoppers. Journal of Agricultural and Food Chemistry 2014, 62, 8997-9001, doi:10.1021/jf502484n
(28) Solfrizzo, M.; Vitti, C.; De Girolamo, A.; Visconti, A.; Logrieco, A.; Fanizzi, F.P. Radicinols and Radicinin Phytotoxins Produced by Alternaria radicina on Carrots. Journal of Agricultural and Food Chemistry 2004, 52, 3655-3660, doi: 10.1021/jf035254t.
(29) Fajardo A, Martinez JL. 2008. Antibiotics as signals that trigger specific bacterial responses. Curr Opin Microbiol 11:161-167. https://doi.org/10 .1016/j.mib.2008.02.006.
(30) Brinkmann, S.; Kurz, M.; Patras, M.A.; Hartwig, C.; Marner, M.; Leis, B.; Billion, A.; Kleiner, Y.; Bauer, A.; Toti, L.; et al. Genomic and chemical decryption of the Bacteroidetes phylum for its potential to biosynthesize natural products. bioRxiv 2021, 2021.2007.2030.454449, doi:10.1101/2021.07.30.454449
(31) Medema MH, Kottmann R, Yilmaz P, Cummings M, Biggins JB, Blin K, de Bruijn I, Chooi YH, Claesen J, Coates RC, Cruz-Morales P, Duddela S, Diisterhus S, Edwards DJ, Fewer DP, Garg N, Geiger C, Gomez-Escribano JP, Greule A, Hadjithomas M, Haines AS, Helfrich EJN, Hillwig ML, Ishida K, Jones AC, Jones CS, Jungmann K, Kegler C, Kim HU, Kötter P, Krug D, Masschelein J, Melnik AV, Mantovani SM, Monroe EA, Moore M, Moss N, Nützmann H-W, Pan G, Pati A, Petras D, Reen FJ, Rosconi F, Rui Z, Tian Z, Tobias NJ, Tsunematsu Y, Wiemann P, Wyckoff E, Yan X, Yim G, Yu F, Xie Y, Aigle B, et al. 2015. Minimum information about a biosynthetic gene cluster. Nat Chem Biol 11:625-631. https://doi.org/10.1038/nchembio.1890.
(32) Reasoner DJ, Geldreich EE. 1985. A new medium for the enumeration and subculture of bacteria from potable water. Appl Environ Microbiol 49:1-7., DOI : 10.1128/aem.49.1.1-7.1985
(33) Marner, M.; Patras, M.A.; Kurz, M.; Zubeil, F.; Förster, F.; Schuler, S.; Bauer, A.; Hammann, P.; Vilcinskas, A.; Schäberle, T.F.; et al. Molecular Networking-Guided Discovery and Characterization of Stechlisins, a Group of Cyclic Lipopeptides from a Pseudomonas sp. Journal of Natural Products 2020, 83, 2607-2617, doi: 10.1021/acs.jnatprod.0c00263.
(34) M. J. Davis, A.H.P., S. V. Thomson. Isolation media for the Pierce's disease bacterium. Phytopathology 1980, 70, 425-429.

## Claims

1. **A method for treating a disease and/or increasing resistance** against an Xylella infection in a plant, wherein said method comprises administering to the plant a plant protection agent that comprises:
a peptide that comprises a chain of amino acids with the sequence X₁X₂X₃X₄X₅X₆X₇X₈XgX₁ₒX₁₁X₁₂X₁₃X₁₄X₁₅;
wherein X is an amino acid or no amino acid (absent) according to the following definitions:
- X₁ is absent or L-Dap;
- X₂ is *N*-methyl-L-Val;
- X₃ is D-*allo*-Thr;
- X₄ is D-Ala;
- X₅ is β-OH-L-*allo*-Asp;
- X₆ is β-OH-L-Phe;
- X₇ is D-*allo*-Ile;
- X₈ is L-Ser;
- X₉ is D-Lys;
- X₁₀ is L-Dap;
- X₁₁ is D-Val, D-*allo*-Ile or D-Leu;
- X₁₂ is β-OH-L-*allo*-Asp;
- X₁₃ is L-Leu;
- X₁₄ is β-OH-L-*allo*-Asp;
- X₁₅ is β-OH-L-Ile;
wherein L-Dap is L-2,3-Diaminopropionic acid;
wherein sequential amino acids are connected by a carboxamide bond, and wherein a fatty acid group R is bound covalently to an amino acid of the chain of amino acids of the peptide; and
wherein the amino acids X₃X₄X₅X₆X₇X_{S}X₉X₁₀X₁₁X₁₂X₁₃X₁₄X₁₅ of the peptide form a cycle and the non-side chain carboxyl group of the amino acid X₁₅ is covalently connected to the amino acid X₃;
or wherein the plant protection agent comprises:
a complex consisting of said peptide and a metal atom;
a nucleic acid sequence encoding for an NRPS able to synthesize the peptide or parts of the peptide; and/or
a cell, wherein the cell comprises the nucleic acid sequence encoding for the NRPS, and/or wherein the cell comprises a vector comprising the nucleic acid sequence encoding for the NRPS.

2. The method of claim 1, wherein the chain of amino acids is:
X₁~X₂~X₃~X₄~X₅~X₆~X₇~X₈~X₉~X₁₀~X₁₁~X₁₂~X₁₃~X₁₄~X₁₅ or
X₁~X₂~X₃~X₄~X₅~X₆~X₇~X₈~X₉~X₁₀~X₁₁~X₁₂~X₁₃~X₁₄~X₁₅,
wherein ~ between two adjacent amino acids indicates that the two adjacent amino acids are covalently connected by a peptide bond connecting the non-side chain carboxyl group of the first of the two adjacent amino acids with the non-side chain amino group of the second of the two adjacent amino acids.
wherein * between two adjacent amino acids indicates that the two adjacent amino acids are covalently connected by a isopeptide bond connecting the non-side chain carboxy group of the first of the two adjacent amino acid with a side chain amino group of the second of the two adjacent amino acids.

3. The method of any one of claims 1 or 2, wherein the fatty acid group R is selected from a compound with the structure (I) 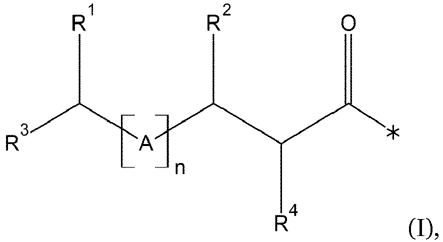
wherein each R¹, R³, and R⁴ is independently selected from the group consisting of: H, C₁₋₆ alkyl, C₂₋₆ alkenyl, C₂₋₆ alkynyl and 3 to 6 membered cycloalkyl, wherein each H may independently be substituted by Cl and/or F, preferably wherein each R₁ is independently selected from the group -H, -CH₃, *tert*-butyl, isobutyl and cyclopropyl;
wherein R² is selected from the group consisting of -H, -CN, azido, -NO₂, -NH₂, - OH, -OCH₃₋ₓFₓ, -SH, -SCH₃₋ₓFₓ, -CH₃₋ₓFₓ (wherein x is 0 to 3), 3 to 6 membered cycloalkyl, 3 to 6 membered heterocyclyl, 3 to 6 membered aryl, and 3 to 6 membered heteroaryl, wherein each hydrogen atom in the 3 to 6 membered cycloalkyl, 3 to 6 membered heterocyclyl, 3 to 6 membered aryl or 3 to 6 membered heteroaryl may independently be substituted by a substituent selected from the group of -CN, halogen, (preferably wherein the halogen is F and/or Cl), azido, -NO₂, -NH₂, -OH, -OCH₃₋ₓFₓ, - SH, -SCH₃₋ₓFₓ, and -CH₃₋ₓFₓ, wherein x is 0 to 3, preferably wherein the heteroaryl or heterocycyl contains at least one heteroatom selected from the group of nitrogen, oxygen, and/or sulphur;
wherein each of the A is independently selected from the group of -(CH₂)-, -(CH=CH)-, - (C=C)-, and -(C=O)-, wherein each hydrogen atom of -(CH₂)- and -(CH=CH)- may independently be substituted with halogen, (preferably wherein the halogen is Cl, and/or F), and/or CH₃₋ₓFₓ, wherein x is 0 to 3; and
wherein n is 0 to 20,

4. The method of claim 3, wherein
n is 5;
R¹, R³, and R⁴ are each independently selected from -CH₃ or -H;
R² is -NH₂ or -OH; and
A is -(CH₂)-.

5. The method of any one of claims 1 to 4, wherein the fatty acid group R is selected from a compound with the structure 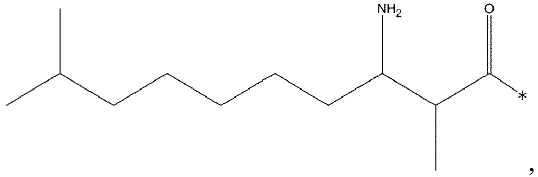 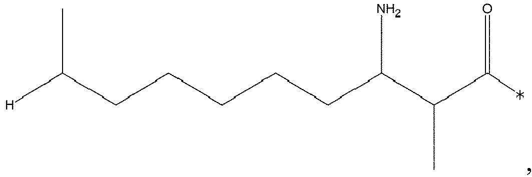 or

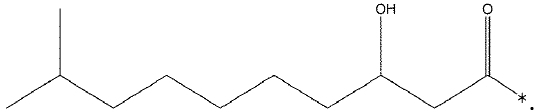

6. The method of any one of claims 1 to 5, wherein the fatty acid group R is bound to the amino acid of the chain of amino acids at the X position with the lowest integer.

7. The method of any one of claims 1 to 6, wherein the fatty acid group R is bound to the chain of amino acids via a carboxamide bond.

8. The method of any one of claims 1 to 7, wherein the peptide is any one of the molecules with the structure (II-VII)

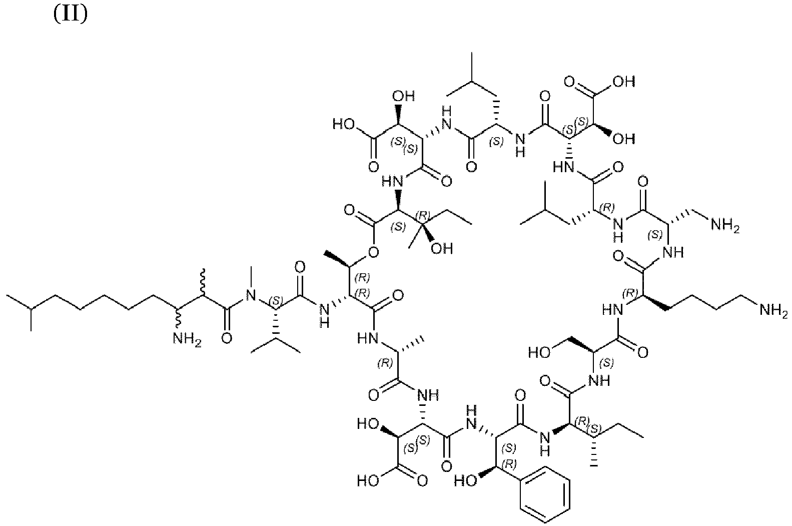

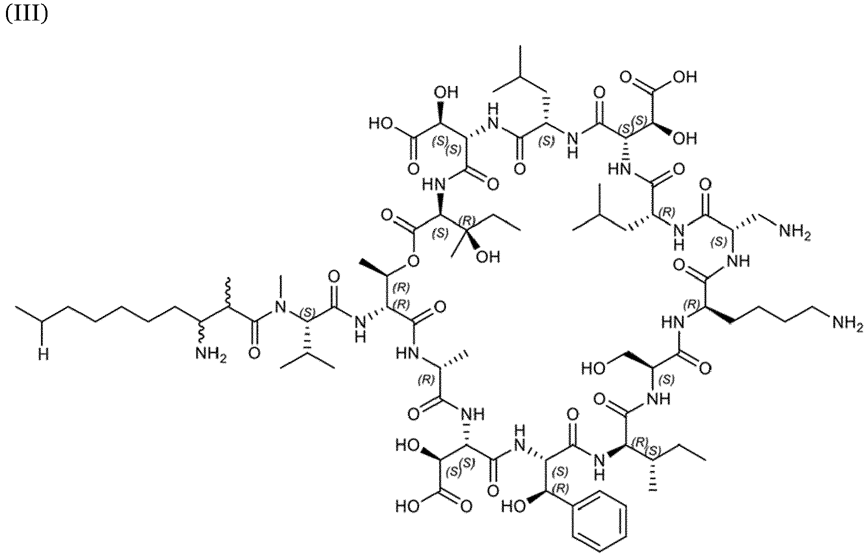

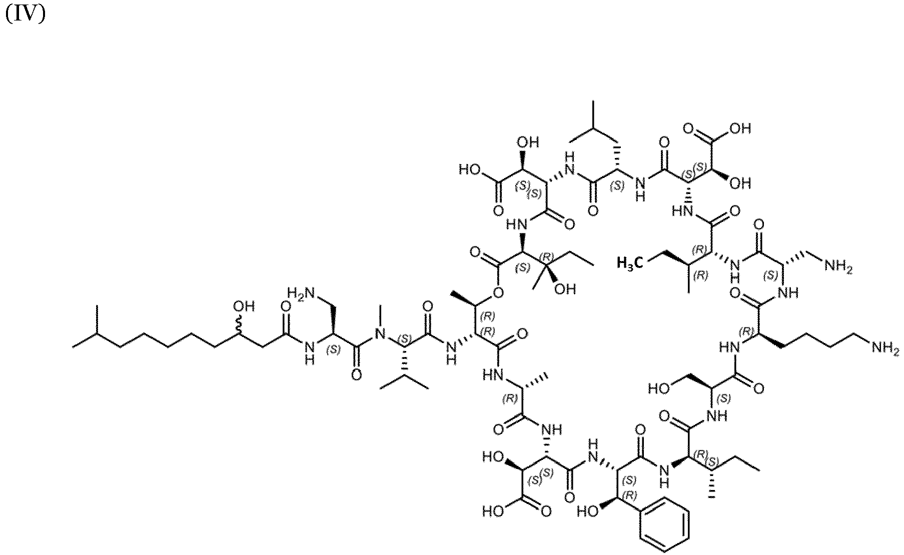

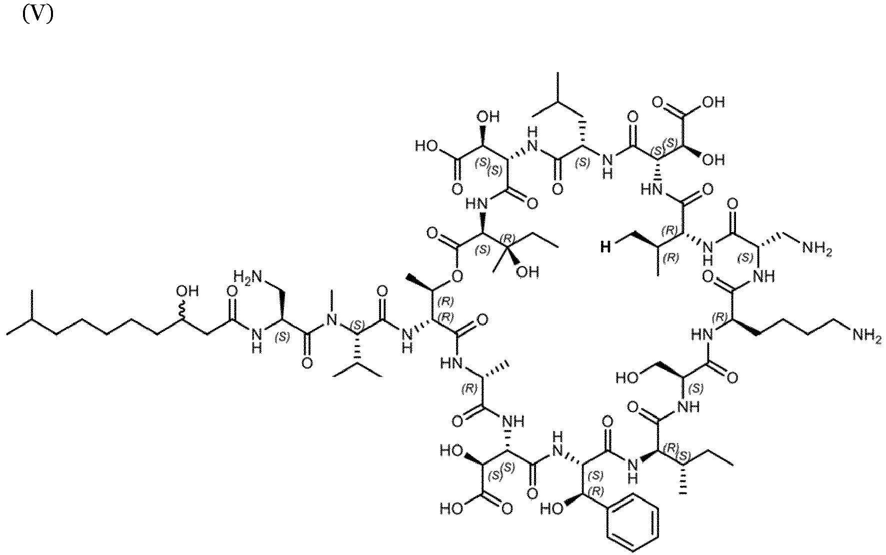

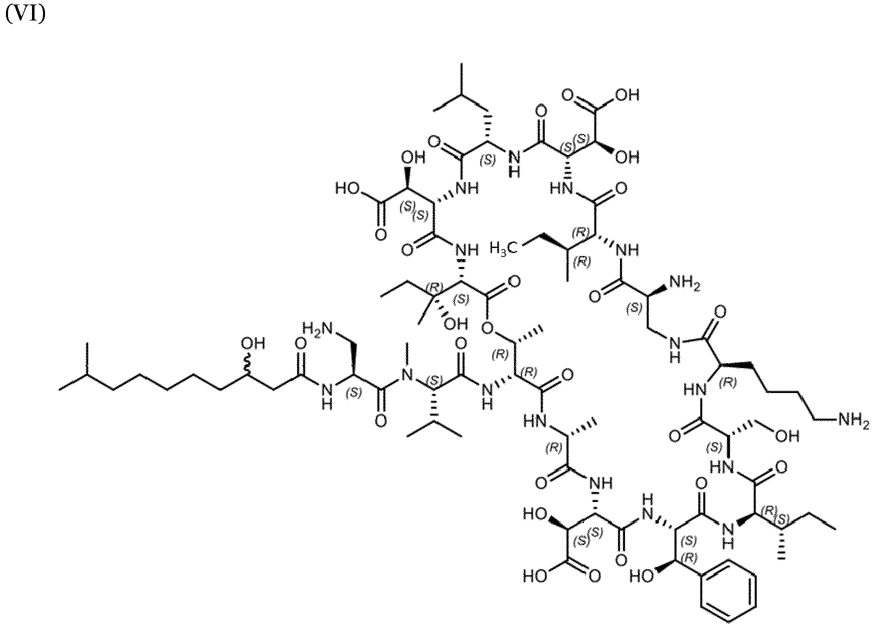

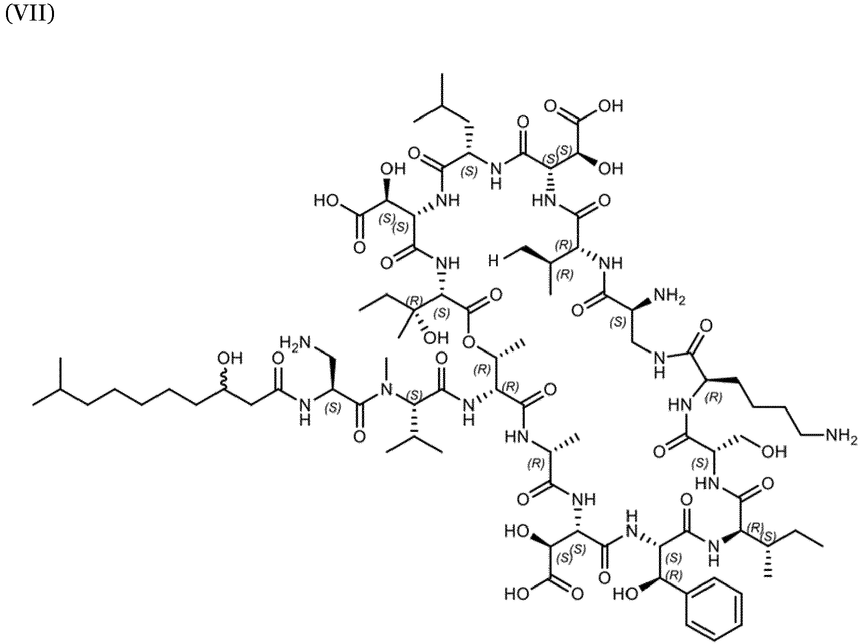

9. The method of any one of claims 1 to 8, wherein the complex consists of the peptide and a metal atom selected from the group of Fe, Cu, and Zn, preferably Fe.

10. The method of any one of claims 1 to 9, wherein administering comprises applying the plant protection agent directly to the plant and/or applying the plant protection agent to the environment surrounding the plant and/or wherein the plant protection agent is administered into the plant, preferably into the xylem vessel.

11. **Use of a plant protection agent,** in the treatment of a disease and/or increasing resistance against a *Xylella* infection in a plant, wherein the plant protection agent comprises
a peptide that comprises a chain of amino acids with the sequence X₁X₂X₃X₄X₅X₆X₇X₈X₉X₁₀X₁₁X₁₂X₁₃X₁₄X₁₅,
wherein X is an amino acid or no amino acid (absent) according to the following definitions:
- X₁ is absent or L-Dap;
- X₂ is *N*-methyl-L-Val;
- X₃ is D-*allo*-Thr;
- X₄ is D-Ala;
- X₅ is β-OH-L-*allo*-Asp;
- X₆ is β-OH-L-Phe;
- X₇ is D-*allo*-Ile;
- X₈ is L-Ser;
- X₉ is D-Lys;
- X₁₀ is L-Dap;
- X₁₁ is D-Val, D-*allo*-Ile or D-Leu;
- X₁₂ is β-OH-L-*allo*-Asp;
- X₁₃ is L-Leu;
- X₁₄ is β-OH-L-*allo*-Asp;
- X₁₅ is β-OH-L-Ile;
wherein L-Dap is L-2,3-Diaminopropionic acid;
wherein sequential amino acids are connected by a carboxamide bond, and wherein a fatty acid group R is bound covalently to an amino acid of the chain of amino acids of the peptide;
wherein the amino acids X₃X₄X₅X₆X₇X_{S}X₉X₁₀X₁₁X₁₂X₁₃X₁₄X₁₅ of the peptide form a cycle and the non-side chain carboxyl group of the amino acid X₁₅ is covalently connected to the amino acid X₃;
or wherein the plant protection agent comprises;
a complex consisting of said peptide and a metal atom;
a nucleic acid sequence encoding for an NRPS able to synthesize the peptide or parts of the peptide and/or
a cell, wherein the cell comprises the nucleic acid sequence encoding for the NRPS, and/or wherein the cell comprises a vector comprising the nucleic acid sequence encoding for the NRPS.

12. **A method of manufacturing** a peptide that comprises a chain of amino acids with the sequence X₁X₂X₃X₄X₅X₆X₇X₈X₉X₁₀X₁₁X₁₂X₁₃X₁₄X₁₅,
wherein X is an amino acid or no amino acid (absent) according to the following definitions:
- X₁ is absent or L-Dap;
- X₂ is *N*-methyl-L-Val;
- X₃ is D-*allo*-Thr;
- X₄ is D-Ala;
- X₅ is β-OH-L-*allo*-Asp;
- X₆ is β-OH-L-Phe;
- X₇ is D-*allo*-Ile;
- X₈ is L-Ser;
- X₉ is D-Lys;
- X₁₀ is L-Dap;
- X₁₁ is D-Val, D-*allo*-Ile or D-Leu;
- X₁₂ is β-OH-L-*allo*-Asp;
- X₁₃ is L-Leu;
- X₁₄ is β-OH-L-*allo*-Asp;
- X₁₅ is β-OH-L-Ile;
wherein L-Dap is L-2,3-Diaminopropionic acid;
wherein sequential amino acids are connected by a carboxamide bond, and wherein a fatty acid group R is bound covalently to an amino acid of the chain of amino acids of the peptide;
wherein the amino acids X₃X₄X₅X₆X₇X_{S}X₉X₁₀X₁₁X₁₂X₁₃X₁₄X₁₅ of the peptide form a cycle and the non-side chain carboxyl group of the amino acid X₁₅ is covalently connected to the amino acid X₃;
wherein the method comprises a recombinant and/or a chemical synthesis.

13. **A cell,** comprising a recombinant nucleic acid sequence and/or comprising a recombinant vector comprising a nucleic acid sequence encoding for a peptide, wherein the peptide comprises a chain of amino acids with the sequence X₁X₂X₃X₄X₅X₆X₇X₈X₉X₁₀X₁₁X₁₂X₁₃X₁₄X₁₅;
wherein X is an amino acid or no amino acid (absent) according to the following definitions:
- X₁ is absent or L-Dap,
- X₂ is *N*-methyl-L-Val
- X₃ is D-*allo*-Thr
- X₄ is D-Ala
- X₅ is β-OH-L-*allo*-Asp
- X₆ is β-OH-L-Phe
- X₇ is D-*allo*-Ile
- X₈ is L-Ser
- X₉ is D-Lys
- X₁₀ is L-Dap
- X₁₁ is D-Val, D-*allo*-Ile or D-Leu
- X₁₂ is β-OH-L-*allo*-Asp
- X₁₃ is L-Leu
- X₁₄ is β-OH-L-*allo*-Asp
- X₁₅ is β-OH-L-Ile
wherein L-Dap is L-2,3-Diaminopropionic acid,
wherein sequential amino acids are connected by a carboxamide bond, and wherein a fatty acid group R is bound covalently to an amino acid of the chain of amino acids of the peptide
wherein the amino acids X₃X₄X₅X₆X₇X_{S}X₉X₁₀X₁₁X₁₂X₁₃X₁₄X₁₅ of the peptide form a cycle and the non-side chain carboxyl group of the amino acid X₁₅ is covalently connected to the amino acid X₃.

14. A **plant protection** composition comprising at least one peptide or a salt thereof as defined in any one of claims 1 to 13, and a phytopharmaceutically acceptable carrier and/or excipient.

15. **Use of a peptide** as defined in any one of claims 1 to 13 as a plant protection agent against *Xylella.*
